(19) Europäisches Patentamt
European Patent Office
Office européen des brevets

(11) **EP 4 682 160 A1**

(12) **EUROPEAN PATENT APPLICATION**
published in accordance with Art. 153(4) EPC

(43) Date of publication:
**21.01.2026 Bulletin 2026/04**

(21) Application number: **23878790.7**

(22) Date of filing: **15.08.2023**

(51) International Patent Classification (IPC):
*C07K 14/165* (2006.01)     *C07K 19/00* (2006.01)
*C12N 15/50* (2006.01)     *A61K 39/215* (2006.01)
*A61P 31/14* (2006.01)

(52) Cooperative Patent Classification (CPC):
Y02A 50/30

(86) International application number:
**PCT/CN2023/113170**

(87) International publication number:
**WO 2024/082795 (25.04.2024 Gazette 2024/17)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **17.03.2023 CN 202310260672**

(71) Applicant: **WestVac Biopharma Co., Ltd.
Chengdu, Sichuan 610000 (CN)**

(72) Inventors:
• **WEI, Xiawei
  Chengdu, Sichuan 610065 (CN)**
• **LU, Guangwen
  Chengdu, Sichuan 610065 (CN)**
• **CHENG, Ping
  Chengdu, Sichuan 610065 (CN)**
• **YANG, Li
  Chengdu, Sichuan 610065 (CN)**
• **LI, Jiong
  Chengdu, Sichuan 610065 (CN)**

• **WANG, Wei
  Chengdu, Sichuan 610065 (CN)**
• **YANG, Jingyun
  Chengdu, Sichuan 610065 (CN)**
• **WEI, Yuquan
  Chengdu, Sichuan 610065 (CN)**
• **WANG, Zhenling
  Chengdu, Sichuan 610065 (CN)**
• **SHEN, Guobo
  Chengdu, Sichuan 610065 (CN)**
• **YANG, Jinliang
  Chengdu, Sichuan 610065 (CN)**
• **ZHAO, Zhiwei
  Chengdu, Sichuan 610065 (CN)**

(74) Representative: **Gulde & Partner
Patent- und Rechtsanwaltskanzlei mbB
Berliner Freiheit 2
10785 Berlin (DE)**

Remarks:
The complete document including Reference
Table(s) and the Sequence Listing(s) can be
downloaded from the EPO website

(54) **PROTEIN AND VACCINE AGAINST INFECTIONS OF SARS-COV-2 OMICRON MUTANT STRAIN XBB AND SUBTYPE THEREOF**

(57) The present invention relates to a protein and a vaccine against infections by a SARS-CoV-2 Omicron variant XBB and subvariants thereof, which belongs to the medicine field. To address the lack of effective prophylactic and therapeutic agents against the infections caused by SARS-CoV-2 Omicron variant XBB and subvariants thereof, the present invention provides proteins and vaccines against infections by the variants, the vaccines are designed based on the full-length S protein, the receptor-binding domain (RBD) sequence and optimized sequences of SARS-CoV-2 Omicron variant XBB and subvariant XBB.1.5, thereof, which are are capable of aiding the host in combating coronavirus infections, and particularly have a relatively good preventive and therapeutic effect against cross-infections caused by SARS-CoV-2 Omicron variant XBB and subvariants thereof.

FIG. 1

**Description**

**TECHNICAL FIELD**

The present invention relates to a protein and a vaccine against infections by a SARS-CoV-2 Omicron variant XBB and subvariants thereof, and belongs to the medicine field.

**BACKGROUND**

The novel coronavirus (SARS-Cov-2) is a new β coronavirus named by the World Health Organization. This virus is enveloped, and its particles are round or oval, often polymorphic, with a diameter of 60nm-140nm. Its genetic characteristics are significantly different from those of SARS-CoV and MERS-CoV, so the virus is a branch of novel coronavirus that has not been discovered in humans before. At present, there are five major types of mutated novel coronavirus: Alpha, Beta, Gamma, Delta, and Omicron, and the Omicron variants are further divided into several subvariants, such as BA.1, BA.2, BA.2.12.1, BA.4, BA.5, BQ.1.1, and XBB.1.5.

Main structural proteins of SARS-CoV-2 include spike (Spike, S) protein, envelop (Envelop, E) protein, membrane (Membrane, M) protein and nucleocapsid (Nucleocapsid, N) protein, among which the S protein plays a key role in virus infection and virulence and is often used as an antigen for vaccines. Because the SARS-CoV-2 mutation variant XBB contains multiple mutation sites, the virus's S protein also contains multiple mutation sites, which causes the variant XBB and its subvariants to escape the antibodies stimulated by the vaccine of the mutant strains of the new coronavirus (Alpha, Beta, Gamma, Delta, and Omicron), resulting in the failure of the new coronavirus vaccine or reduced protection, which brings great pressure to the prevention and control of novel coronavirus. Therefore, it is very important to develop vaccines against the SARS-CoV-2 virus mutation variant XBB and its subvariants, especially broad-spectrum vaccines for various mutant SARS-CoV-2 viruses, for the prevention and treatment of SARS-CoV-2.

**SUMMARY**

The present invention aims at solving at least one of the technical problems existing in the existing technology. Therefore, the objective of the present invention is to provide a protein against infection by a SARS-CoV-2 Omicron variant XBB and subvariants thereof. The present invention also provides a vaccine for preventing and/or treating the infections caused by SARS-CoV-2 Omicron variant XBB and subvariants thereof, including a recombinant protein vaccine and an adenovirus vector vaccine containing the protein as described, and also provides a composition or a combined drug containing the two vaccines.

The present invention first provides a protein for resisting the infections caused by SARS-CoV-2 Omicron variant XBB and subvariants thereof, which contains an amino acid sequence selected from any one of SEQ ID NO.1 to SEQ ID NO.7. Preferably, the protein is selected from SEQ ID NO.3 or SEQ ID NO.5.

The present invention further provides a precursor of said protein, which is connected to a signal peptide and/or protein tag on said protein.

Preferably, the protein tag is selected from at least one of a histidine tag (6His tag), a thioredoxin tag (Trx tag), a glutathione transferase tag, a ubiquitin-like modified protein tag, a maltose-binding protein tag, a c-Myc protein tag, an Avi tag protein tag, and a nitrogen source using substance A protein tag.

Further, a protease recognition region for excising the protein tag is also linked to the protein against infection by the SARS-CoV-2 Omicron variant strain XBB and subvariants thereof.

Preferably, the protease is selected from at least one of an enterokinase (EK enzyme), a TEV protease, a thrombin, a blood coagulation factor Xa, a carboxypeptidase A and a rhinovirus 3c protease.

Further, the amino acid sequence of the precursor is selected from at least one of SEQ ID NO.8, SEQ ID NO.10, SEQ ID NO.12, SEQ ID NO.14, SEQ ID NO.16, SEQ ID NO.18, and SEQ ID NO.20. Preferably, the protein sequence is selected from SEQ ID NO.12 or SEQ ID NO.16.

The present invention also provides a polynucleotide, which encodes the protein or the precursor as described.

Further, the nucleotide sequence is selected from at least one of SEQ ID NO.9, SEQ ID NO.11, SEQ ID NO.13, SEQ ID NO.15, SEQ ID NO.17, SEQ ID NO.19, and SEQ ID NO.21. Preferably, the polynucleotide sequence is selected from SEQ ID NO.13 or SEQ ID NO.17.

The present invention further provides a recombinant vector, comprising the polynucleotide.

Further, the recombinant vector adopts at least one of an insect baculovirus expression vector, a mammalian cell expression vector, an Escherichia coli expression vector and a yeast expression vector.

Preferably, the insect baculovirus expression vector is pFastBac1.

Preferably, the Escherichia coli expression vector is pET32a.

Preferably, the yeast expression vector is pPICZaA.

Preferably, the mammalian cell expression vector is a CHO cell expression vector.

More preferably, the CHO cell expression vector is pTT5 or FTP-002.

The present invention further provides a host cell including the recombinant vector.

Further, the host cell adopts at least one of an insect cell, a mammalian cell, Escherichia coli and a yeast.

Preferably, the insect cell is selected from at least one of a sf9 cell, a sf21 cell and a Hi5 cell.

Preferably, the mammalian cell is a CHO cell.

The present invention further provides a preparation method for the protein or the precursor, wherein the method includes the following steps: culturing the host cell to express the protein or precursor, and then recovering the protein.

The present invention also provides a protein composition for resisting infections caused by SARS-CoV-2 Omicron variants and subvariants thereof, which comprises any combination of two or more of BA.5 sequence-1, BA.5 sequence-2, XBB.1.5 sequence-1, XBB.1.5 sequence-2, XBB.1.5 sequence-3, XBB.1.5 sequence-4, and S-XBB.1.5 proteins.

Preferably, the protein composition comprises one of BA.5 sequence-1, BA.5 sequence-2, any one of XBB.1.5 sequence-1, XBB.1.5 sequence-2, XBB.1.5 sequence-3, XBB.1.5 sequence-4 and at least two combinations of, S-XBB.1.5 protein.

More preferably, the protein composition comprises one of BA.5 sequence-1 abd BA.5 sequence-2 and any one of the combination of, XBB.1.5 sequence-3 and XBB.1.5 sequence-4 with S-XBB.1.5 protein.

The present invention further provides a recombinant protein vaccine for preventing and/or treating infection by a SARS-CoV-2 Omicron variant and subvariants thereof, comprising the protein, the precursor and/or the protein composition, as well as a pharmaceutically acceptable excipient or adjuvant component.

Further, the adjuvant component is an immunologic adjuvant.

Preferably, the immunologic adjuvant is selected from at least one of the following: a squalene-based oil-in-water emulsion, an aluminum salt, a calcium salt, a phytosaponin, a phytopolysaccharide, a monophosphate lipid A, a muramyl dipeptide, a muramyl tripeptide, a bacterial toxin, a GM-CSF cytokine, a lipid and a cationic liposomal material.

Further, at least one of the following is satisfied: the squalene oil-in-water emulsion is MF59;

the aluminum salt is selected from at least one of aluminum hydroxide and alum;

the calcium salt is tricalcium phosphate;

the phytosaponin is either QS-21 or ISCOM;

the plant polysaccharide is astragalus polysaccharide;

the bacterial toxin is selected from at least one of recombinant cholera toxin and diphtheria toxin;

the lipid is selected from at least one of the following: phosphatidylethanolamine, phosphatidylcholine, cholesterol, dioleoylphosphatidylethanolamine;

the cationic liposome material is selected from at least one of the following: (2,3-dioleoyloxypropyl) trimethyl ammonium chloride, N-[1-(2,3-dioleoyl chloride) propyl]-N,N,N-trimethylamine chloride, cationic cholesterol, dimethyl-2,3-dioleyloxypropyl-2-(2-spermidinecarboxamido)ethyl ammonium trifluoroacetate, trimethyldodecylammonium bromide, trimethyltetradecylammonium bromide, trimethylhexadecylammonium bromide, dimethyldioctadecylammonium bromide, and CpG ODN.

The present invention also provides an adenovirus vector vaccine against the infections caused by SARS-CoV-2 Omicron variant XBB and subvariants thereof, which constructs a recombinant vector containing a polynucleotide encoding the amino acid sequence of the protein as described, the precursor or the protein composition as described.

Preferably, the nucleotide sequence of the polynucleotide is selected from at least one of SEQ ID NO.9, SEQ ID NO.11, SEQ ID NO.13, SEQ ID NO.15, SEQ ID NO.17, SEQ ID NO.19, and SEQ ID NO.21. More preferably, the polynucleotide sequence contained in the adenovirus vector is selected from the polynucleotide for constructing S-XBB.1.5, as shown in SEQ ID NO.21.

Further, the adenovirus vector is selected from at least one of the following: an Adenovirus, an Ankara vaccinia virus, and an adeno-associated virus.

Preferably, it is selected from a human type 5, type 35 or type 26 and/or chimpanzee AdC68 or AdC7 replication-deficient adenovirus.

More preferably, it is selected from a human type 5 replication-deficient adenovirus with combined deletion of E1 and E3.

The present invention provides a preparation method for the adenovirus in the adenovirus vector vaccine as described, comprising the following steps: constructing a shuttle plasmid vector of the polynucleotide as described; then transfecting the constructed shuttle plasmid vector into host cells together with the backbone plasmid, and culturing the host cells; obtaining replication-deficient recombinant adenovirus, and then expanding the culture and purifying.

Further, the adenovirus vector vaccine also includes a pharmaceutically acceptable adjuvant, vector, diluent or excipient.

Further, the recombinant protein vaccine and adenovirus vector vaccine are intradermal or subcutaneous injection preparations, intramuscular injection preparations, intravenous injection preparations, oral or nasal spray preparations.

Preferably, the vaccine is an intramuscular injection preparation and a nasal spray preparation.

The present invention also provides a composition for treating and/or preventing SARS-CoV-2 Omicron variants and subvariants thereof, which is a compound preparation containing the recombinant protein vaccine and the adenovirus vector vaccine as active ingredients.

The present invention provides a combined drug for resisting the infections caused by SARS-CoV-2 Omicron variant XBB and subvariants thereof, which contains the recombinant protein vaccine and the adenovirus vector vaccine, administered separately or simultaneously.

Preferably, the composition or combined drug is a combination or combined drug of recombinant protein vaccine 1, recombinant protein vaccine 2 and adenovirus vector vaccine.

More preferably, the recombinant protein vaccine 1 contains at least one amino acid sequence of BA.5 sequence-1, BA.5 sequence-2 protein or precursor; the recombinant protein vaccine 2 contains at least one amino acid sequence of XBB.1.5 sequence-1, XBB.1.5 sequence-2, XBB.1.5 sequence-3, XBB.1.5 sequence-4 protein or precursor; the adenovirus vector vaccine contains the polynucleotide sequence of S-XBB.1.5.

Further, the amino acid sequence contained in the recombinant protein vaccine 1 is selected from at least one of SEQ ID NO.1, SEQ ID NO.2, SEQ ID NO.8, and SEQ ID NO.10.

Further, the amino acid sequence contained in the recombinant protein vaccine 2 is selected from at least one of SEQ ID NO.3 to SEQ ID NO.6, SEQ ID NO.12, SEQ ID NO.14, SEQ ID NO.16, and SEQ ID NO.18. Preferably, it is selected from at least one of SEQ ID NO.5, SEQ ID NO.6, SEQ ID NO.16, and SEQ ID NO.18.

Further, the adenovirus vector vaccine contains a polynucleotide sequence as shown in SEQ ID NO.21.

Further, the composition or combined drug is an intradermal or subcutaneous injection preparation, an intramuscular injection preparation, an intravenous injection preparation, an oral or nasal spray preparation; preferably, the vaccine is intramuscular injection preparation and a nasal spray preparation.

The present invention also provides the use of the protein as described, the precursor as described, the protein composition as described, the recombinant protein vaccine as described, the adenovirus vector vaccine, the vaccine composition or the combined drug in the preparation of a drug for treating and/or preventing infection or pathogenicity caused by SARS-CoV-2 Omicron variants and subvariants thereof.

The present invention also provides a vaccine composition for treating and/or preventing infections caused by SARS-CoV-2 Omicron variants and subvariants thereof, which contains a recombinant protein vaccine and an adenovirus vector vaccine; the amino acid sequence of the recombinant protein vaccine is selected from at least one of SEQ ID NO.1 to SEQ ID NO.7, SEQ ID NO.8 to SEQ ID NO.20; the nucleotide sequence for the antigen of the adenoviral vector vaccine is selected from SEQ ID NO.23.

The present invention also provides a combined drug for treating and/or preventing infections caused by SARS-CoV-2 Omicron variants and subvariants thereof, and the above-mentioned recombinant protein vaccine and adenovirus vector vaccine shall be administered separately or simultaneously; the amino acid sequence of the recombinant protein vaccine is selected from at least one of SEQ ID NO.1 to SEQ ID NO.7, SEQ ID NO.8 to SEQ ID NO.20; the nucleotide sequence for the antigen of the adenoviral vector vaccine is selected from SEQ ID NO.23.

The following sequences are constructed by the applicant based on the optimized RBD sequences in the S protein of SARS-CoV-2 mutant Omicron variants BA.5, XBB and subvariants XBB.1.5. and the constructed proteins or precursors are defined as BA.5 sequence-1, BA.5 sequence-2, XBB.1.5 sequence-1, XBB.1.5 sequence-2, XBB.1.5 sequence-3, XBB.1.5 sequence-4, S-XBB.1.5 protein or precursor.

SEQ ID NO.1 BA.5 sequence-1

VQPTESIVRFPNITNLCPFDEVFNATRFASVYAWNRKRISNCVADYSVLYNFAPFFAF
KCYGVSPTKLNDLCFTNVYADSFVIRGNEVSQIAPGQTGNIADYNYKLPDDFTGCVIAW
NSNKLDSKVGGNYNYRYRLFRKSNLKPFERDISTEIYQAGNKPCNGVAGVNCYFPLQSY
GFRPTYGVGHQPYRVVVLSFELLHAPATVCGPKKSTNLVKNKSVNFNFNGLYENQKLIA
NQFNSAIGKIQDSLSSTASALGKLQDVVNQNAQALNTLVKQLKNHTSPDVDLGDISGIN
ASVVNIQKEIDRLNEVAKNLNESLIDLQEL

SEQ ID NO.2 BA.5 sequence-2

VQPTESIVRFPNITNLCPFDEVFNATRFASVYAWNRKRISNCVADYSVLYNSASFSAF
KCYGVSPTKLNDLCFTNVYADSFVIRGNEVSQIAPGQTGNIADYNYKLPDDFTGCVIAW
NSNKLDSKVGGNYNYRYRLFRKSNLKPFERDISTEIYQAGNKPCNGVAGVNCYFPLQSY
GFRPTYGVGHQPYRVVVLSFELLHAPATVCGPKKSTNLVKNKSVNFNFNGLYENQKLIA
NQFNSAIGKIQDSLSSTASALGKLQDVVNQNAQALNTLVKQLKNHTSPDVDLGDISGIN
ASVVNIQKEIDRLNEVAKNLNESLIDLQEL

SEQ ID NO.3 XBB.1.5 sequence-1

VQPTESIVRFPNITNLCPFHEVFNATTFASVYAWNRKRISNCVADYSVIYNFAPFFAFK
CYGVSPTKLNDLCFTNVYADSFVIRGNEVSQIAPGQTGNIADYNYKLPDDFTGCVIAWN
SNKLDSKPSGNYNYLYRLFRKSKLKPFERDISTEIYQAGNKPCNGVAGPNCYSPLQSYGF
RPTYGVGHQPYRVVVLSFELLHAPATVCGPKKSTNLVKNKCVNFNFNG

SEQ ID NO.4 XBB.1.5 sequence-2

VQPTESIVRFPNITNLCPFHEVFNATTFASVYAWNRKRISNCVADYSVIYNSASFSAF
KCYGVSPTKLNDLCFTNVYADSFVIRGNEVSQIAPGQTGNIADYNYKLPDDFTGCVIAW
NSNKLDSKPSGNYNYLYRLFRKSKLKPFERDISTEIYQAGNKPCNGVAGPNCYSPLQSYG
FRPTYGVGHQPYRVVVLSFELLHAPATVCGPKKSTNLVKNKCVNFNFNG

SEQ ID NO.5 XBB.1.5 sequence-3

VQPTESIVRFPNITNLCPFHEVFNATTFASVYAWNRKRISNCVADYSVIYNFAPFFAFK
CYGVSPTKLNDLCFTNVYADSFVIRGNEVSQIAPGQTGNIADYNYKLPDDFTGCVIAWN
SNKLDSKPSGNYNYLYRLFRKSKLKPFERDISTEIYQAGNKPCNGVAGPNCYSPLQSYGF
RPTYGVGHQPYRVVVLSFELLHAPATVCGPKKSTNLVKNKSVNFNFNGLYENQKLIANQ
FNSAIGKIQDSLSSTASALGKLQDVVNQNAQALNTLVKQLKNHTSPDVDLGDISGINASV
VNIQKEIDRLNEVAKNLNESLIDLQEL

SEQ ID NO.6 XBB.1.5 sequence-4

VQPTESIVRFPNITNLCPFHEVFNATTFASVYAWNRKRISNCVADYSVIYNSASFSAF
KCYGVSPTKLNDLCFTNVYADSFVIRGNEVSQIAPGQTGNIADYNYKLPDDFTGCVIAW
NSNKLDSKPSGNYNYLYRLFRKSKLKPFERDISTEIYQAGNKPCNGVAGPNCYSPLQSYG
FRPTYGVGHQPYRVVVLSFELLHAPATVCGPKKSTNLVKNKSVNFNFNGLYENQKLIAN
QFNSAIGKIQDSLSSTASALGKLQDVVNQNAQALNTLVKQLKNHTSPDVDLGDISGINAS
VVNIQKEIDRLNEVAKNLNESLIDLQEL

SEQ ID NO.7 S-XBB.1.5

VNLITRTQSYTNSFTRGVYYPDKVFRSSVLHSTQDLFLPFFSNVTWFHAIHVSGTNG
TKRFDNPALPFNDGVYFASTEKSNIIRGWIFGTTLDSKTQSLLIVNNATNVVIKVCEFQFC
NDPFLDVYQKNNKSWMESEFRVYSSANNCTFEYVSQPFLMDLEGKEGNFKNLREFVFK
NIDGYFKIYSKHTPINLERDLPQGFSALEPLVDLPIGINITRFQTLLALHRSYLTPVDSSSG
WTAGAAAYYVGYLQPRTFLLKYNENGTITDAVDCALDPLSETKCTLKSFTVEKGIYQTS
NFRVQPTESIVRFPNITNLCPFHEVFNATTFASVYAWNRKRISNCVADYSVIYNFAPFFAFK
CYGVSPTKLNDLCFTNVYADSFVIRGNEVSQIAPGQTGNIADYNYKLPDDFTGCVIAWN
SNKLDSKPSGNYNYLYRLFRKSKLKPFERDISTEIYQAGNKPCNGVAGPNCYSPLQSYGF
RPTYGVGHQPYRVVVLSFELLHAPATVCGPKKSTNLVKNKCVNFNFNGLTGTGVLTESN
KKFLPFQQFGRDIADTTDAVRDPQTLEILDITPCSFGGVSVITPGTNTSNQVAVLYQGVNC
TEVPVAIHADQLTPTWRVYSTGSNVFQTRAGCLIGAEYVNNSYECDIPIGAGICASYQTQ
TKSHRRARSVASQSIIAYTMSLGAENSVAYSNNSIAIPTNFTISVTTEILPVSMTKTSVDCT
MYICGDSTECSNLLLQYGSFCTQLKRALTGIAVEQDKNTQEVFAQVKQIYKTPPIKYFGG
FNFSQILPDPSKPSKRSFIEDLLFNKVTLADAGFIKQYGDCLGDIAARDLICAQKFNGLTV

LPPLLTDEMIAQYTSALLAGTITSGWTFGAGAALQIPFAMQMAYRFNGIGVTQNVLYEN
QKLIANQFNSAIGKIQDSLSSTASALGKLQDVVNHNAQALNTLVKQLSSKFGAISSVLND
ILSRLDKVEAEVQIDRLITGRLQSLQTYVTQQLIRAAEIRASANLAATKMSECVLGQSKR
VDFCGKGYHLMSFPQSAPHGVVFLHVTYVPAQEKNFTTAPAICHDGKAHFPREGVFVSN
GTHWFVTQRNFYEPQIITTDNTFVSGNCDVVIGIVNNTVYDPLQPELDSFKEELDKYFKN
HTSPDVDLGDISGINASVVNIQKEIDRLNEVAKNLNESLIDLQELGKYEQYIKWPWYIWL
GFIAGLIAIVMVTIMLCCMTSCCSCLKGCCSCGSCCKFDEDDSEPVLKGVKLHYT

In order to assist the secretory expression of the protein, a signal peptide has been added to its amino acids when constructing the protein to assist the secretory expression of the protein. Simultaneously, in order to facilitate purification, a His tag has also been added to the amino acid sequence of the protein. The complete BA.5 sequence-1, BA.5 sequence-2, XBB.1.5 sequence-1, XBB.1.5 sequence-2, XBB.1.5 sequence-3, XBB.1.5 sequence-4, and S-XBB.1.5 amino acid sequences of the present invention are shown as SEQ ID NO.8, SEQ ID NO.10, SEQ ID NO.12, SEQ ID NO.14, SEQ ID NO.16, SEQ ID NO.18, and SEQ ID NO.20 respectively.

Further, the corresponding nucleotide sequences encoding the amino acid sequences are shown as SEQ ID NO.9, SEQ ID NO.11, SEQ ID NO.13, SEQ ID NO.15, SEQ ID NO.17, SEQ ID NO.19, and SEQ ID NO.21 respectively.

SEQ ID NO.8 Complete BA.5 sequence-1 signal peptide-Trx tag-6His tag-EK restriction site-RBD sequence-HR1 sequence-HR2 sequence

MLLVNQSHQGFNKEHTSKMVSAIVLYVLLAAAAHSAFAADSIHIKDSDDLKNRLAE
AGDKLVVIDFMATWCGPCKMIGPKLDEMANEMSDSIVVLKVDVDECEDIATEYNINSMP
TFVFVKNSKKIEEFSGANVDKLRNTIIKLKLAGSGSGHMHHHHHHSSGDDDDKVQPTES
IVRFPNITNLCPFDEVFNATRFASVYAWNRKRISNCVADYSVLYNFAPFFAFKCYGVSPTK
LNDLCFTNVYADSFVIRGNEVSQIAPGQTGNIADYNYKLPDDFTGCVIAWNSNKLDSKV
GGNYNYRYRLFRKSNLKPFERDISTEIYQAGNKPCNGVAGVNCYFPLQSYGFRPTYGVG
HQPYRVVVLSFELLHAPATVCGPKKSTNLVKNKSVNFNFNGLYENQKLIANQFNSAIGKI
QDSLSSTASALGKLQDVVNQNAQALNTLVKQLKNHTSPDVDLGDISGINASVVNIQKEI
DRLNEVAKNLNESLIDLQEL

SEQ ID NO. 9 - nucleotide sequence encoding SEQ ID NO. 8

ATGCTGCTGGTGAACCAGAGCCACCAGGGTTTCAACAAGGAACACACTAGCAA

GATGGTGTCTGCTATCGTCCTCTACGTGCTGCTCGCCGCCGCTGCTCACTCAGCTTTC

GCCGCTGACTCTATCCACATCAAGGACTCCGACGACCTCAAGAACCGTTTGGCCGAA

GCCGGCGACAAGCTGGTCGTTATCGACTTCATGGCTACCTGGTGTGGCCCTTGCAAGA

TGATCGGACCTAAGCTGGACGAAATGGCTAACGAGATGAGCGACAGCATCGTCGTTC

TGAAGGTGGACGTCGACGAATGTGAGGACATCGCCACTGAGTACAACATCAACAGCA

TGCCTACCTTCGTTTTCGTGAAGAACTCTAAGAAGATCGAGGAGTTCTCTGGTGCCA

ACGTGGACAAGCTCCGTAACACCATCATCAAGCTCAAGCTGGCCGGCAGCGGTAGCG

GTCACATGCACCACCACCACCACCATTCAAGCGGCGACGACGACGACAAGGTGCAG

CCAACCGAATCAATCGTCCGCTTCCCAAACATCACTAACCTGTGCCCATTCGACGAGG

TGTTCAACGCCACCCGTTTCGCCTCTGTCTACGCTTGGAACCGCAAGCGCATCTCCAA

CTGTGTCGCCGACTACAGCGTGCTGTACAACTTCGCTCCTTTCTTCGCATTCAAGTGT

TACGGAGTGTCCCCTACCAAGCTCAACGATTTGTGCTTCACAAACGTGTACGCTGAC

AGCTTCGTGATCCGTGGTAACGAGGTTTCTCAAATCGCCCCTGGTCAGACAGGTAAC

ATCGCTGACTACAACTACAAGCTCCCAGACGACTTCACTGGCTGTGTTATCGCTTGGA

ACTCAAACAAGCTGGACTCTAAGGTGGGTGGTAACTACAACTACCGGTACCGTCTGT

TCCGCAAGAGCAACCTCAAGCCATTCGAACGTGACATCAGCACAGAGATCTACCAAG

CCGGTAACAAGCCCTGCAACGGCGTTGCCGGAGTCAACTGCTACTTCCCCCTCCAGT

CCTACGGATTCCGCCCAACCTACGGCGTGGGTCACCAGCCATACCGCGTGGTTGTGCT

GAGCTTCGAACTGCTGCACGCCCCCGCTACCGTGTGTGGCCCTAAGAAGAGCACTAA

CCTCGTTAAGAACAAGTCCGTGAACTTCAACTTCAACGGTCTCTACGAAAACCAGAA

GCTGATCGCTAACCAGTTCAACTCCGCCATCGGCAAGATCCAGGACTCCCTGTCCTCC

ACAGCTTCCGCTCTGGGCAAGCTGCAGGACGTTGTGAACCAAAACGCTCAGGCCCT

GAACACACTGGTGAAGCAACTGAAGAACCACACCAGCCCCGACGTTGACCTGGGTG

ACATCAGCGGTATCAACGCTAGCGTGGTGAACATCCAGAAGGAGATCGACAGGCTGA

ACGAAGTGGCTAAGAACTTGAACGAGAGCCTCATCGACCTGCAGGAACTGTAA

SEQ ID NO.10 Complete BA.5 sequence-2 signal peptide-Trx tag-6His tag-EK restriction site-RBD sequence-HR1 sequence-HR2 sequence

MLLVNQSHQGFNKEHTSKMVSAIVLYVLLAAAAHSAFAADSIHIKDSDDLKNRLAE

EP 4 682 160 A1

AGDKLVVIDFMATWCGPCKMIGPKLDEMANEMSDSIVVLKVDVDECEDIATEYNINSMP
TFVFVKNSKKIEEFSGANVDKLRNTIIKLKLAGSGSGHMHHHHHHSSGDDDDKVQPTES
IVRFPNITNLCPFDEVFNATRFASVYAWNRKRISNCVADYSVLYNSASFSAFKCYGVSPTK
LNDLCFTNVYADSFVIRGNEVSQIAPGQTGNIADYNYKLPDDFTGCVIAWNSNKLDSKV
GGNYNYRYRLFRKSNLKPFERDISTEIYQAGNKPCNGVAGVNCYFPLQSYGFRPTYGVG
HQPYRVVVLSFELLHAPATVCGPKKSTNLVKNKSVNFNFNGLYENQKLIANQFNSAIGKI
QDSLSSTASALGKLQDVVNQNAQALNTLVKQLKNHTSPDVDLGDISGINASVVNIQKEI
DRLNEVAKNLNESLIDLQEL

SEQ ID NO. 11 - nucleotide sequence encoding SEQ ID NO. 10

ATGCTGCTGGTGAACCAGAGCCACCAGGGTTTCAACAAGGAACACACTAGCAA
GATGGTGTCTGCTATCGTCCTCTACGTGCTGCTCGCCGCCGCTGCTCACTCAGCTTTC
GCCGCTGACTCTATCCACATCAAGGACTCCGACGACCTCAAGAACCGTTTGGCCGAA
GCCGGCGACAAGCTGGTCGTTATCGACTTCATGGCTACCTGGTGTGGCCCTTGCAAGA
TGATCGGACCTAAGCTGGACGAAATGGCTAACGAGATGAGCGACAGCATCGTCGTTC
TGAAGGTGGACGTCGACGAATGTGAGGACATCGCCACTGAGTACAACATCAACAGCA
TGCCTACCTTCGTTTTCGTGAAGAACTCTAAGAAGATCGAGGAGTTCTCTGGTGCCA
ACGTGGACAAGCTCCGTAACACCATCATCAAGCTCAAGCTGGCCGGCAGCGGTAGCG
GTCACATGCACCACCACCACCACCATTCAAGCGGCGACGACGACGACAAGGTGCAG
CCAACCGAATCAATCGTCCGCTTCCCAAACATCACTAACCTGTGCCCATTCGACGAGG
TGTTCAACGCCACCCGTTTCGCCTCTGTCTACGCTTGGAACCGCAAGCGCATCTCCAA
CTGTGTCGCCGACTACAGCGTGCTGTACAACTCAGCTTCATTCTCAGCATTCAAGTGT
TACGGAGTGTCCCCTACCAAGCTCAACGATTTGTGCTTCACAAACGTGTACGCTGAC
AGCTTCGTGATCCGTGGTAACGAGGTTTCTCAAATCGCCCCTGGTCAGACAGGTAAC
ATCGCTGACTACAACTACAAGCTCCCAGACGACTTCACTGGCTGTGTTATCGCTTGGA
ACTCAAACAAGCTGGACTCTAAGGTGGGTGGTAACTACAACTACCGGTACCGTCTGT
TCCGCAAGAGCAACCTCAAGCCATTCGAACGTGACATCAGCACAGAGATCTACCAAG
CCGGTAACAAGCCCTGCAACGGCGTTGCCGGAGTCAACTGCTACTTCCCCCTCCAGT
CCTACGGATTCCGCCCAACCTACGGCGTGGGTCACCAGCCATACCGCGTGGTTGTGCT
GAGCTTCGAACTGCTGCACGCCCCCGCTACCGTGTGTGGCCCTAAGAAGAGCACTAA

CCTCGTTAAGAACAAGTCCGTGAACTTCAACTTCAACGGTCTCTACGAAAACCAGAA

GCTGATCGCTAACCAGTTCAACTCCGCCATCGGCAAGATCCAGGACTCCCTGTCCTCC

ACAGCTTCCGCTCTGGGCAAGCTGCAGGACGTTGTGAACCAAAACGCTCAGGCCCT

GAACACACTGGTGAAGCAACTGAAGAACCACACCAGCCCCGACGTTGACCTGGGTG

ACATCAGCGGTATCAACGCTAGCGTGGTGAACATCCAGAAGGAGATCGACAGGCTGA

ACGAAGTGGCTAAGAACTTGAACGAGAGCCTCATCGACCTGCAGGAACTGTAA

SEQ ID NO.12 Complete XBB.1.5 sequence-1 signal peptide-Trx tag-6His tag-EK restriction site-RBD sequence

MLLVNQSHQGFNKEHTSKMVSAIVLYVLLAAAAHSAFAADSIHIKDSDDLKNRLAE

AGDKLVVIDFMATWCGPCKMIGPKLDEMANEMSDCIVVLKVDVDECEDIATEYNINSM

PTFVFVKNSKKIEEFSGANVDKLRNTIIKLKLAGSGSGHMHHHHHHSSGDDDDKVQPTE

SIVRFPNITNLCPFHEVFNATTFASVYAWNRKRISNCVADYSVIYNFAPFFAFKCYGVSPTK

LNDLCFTNVYADSFVIRGNEVSQIAPGQTGNIADYNYKLPDDFTGCVIAWNSNKLDSKPS

GNYNYLYRLFRKSKLKPFERDISTEIYQAGNKPCNGVAGPNCYSPLQSYGFRPTYGVGH

QPYRVVVLSFELLHAPATVCGPKKSTNLVKNKCVNFNFNG

SEQ ID NO.13 Nucleotide sequence encoding SEQ ID NO.12

ATGCTACTAGTAAATCAGTCACACCAAGGTTTCAACAAGGAACACACCAGCAAG

ATGGTGAGCGCTATCGTGCTGTACGTCCTGCTGGCCGCTGCTGCTCACAGCGCTTTCG

CTGCTGACTCCATCCACATCAAGGACAGCGACGACCTGAAGAACCGTCTGGCCGAG

GCCGGTGACAAGCTGGTCGTCATCGACTTCATGGCCACTTGGTGCGGTCCTTGCAAG

ATGATCGGCCCTAAGCTGGACGAGATGGCTAACGAGATGTCCGACTGCATCGTGGTC

CTGAAGGTGGACGTCGACGAGTGCGAGGACATCGCCACCGAATACAACATCAACAG

CATGCCCACCTTCGTGTTCGTGAAGAACAGCAAGAAGATCGAGGAATTTTCCGGCGC

TAACGTCGACAAGCTGCGTAACACCATCATCAAGCTGAAGCTGGCCGGCTCCGGCTC

CGGCCACATGCATCACCACCACCACCATTCCTCCGGTGACGACGACGACAAGGTGCA

GCCAACCGAATCTATCGTCAGATTCCCAAACATCACTAACCTGTGCCCTTTCCACGAG

GTGTTCAACGCTACCACTTTCGCCAGCGTCTACGCTTGGAACCGCAAGCGTATCAGC

AACTGCGTCGCCGACTACTCTGTGATCTACAACTTCGCTCCTTTCTTCGCTTTCAAGT

GCTACGGCGTGTCACCTACCAAGCTGAACGACCTGTGCTTCACTAACGTCTACGCCG

11

ACTCCTTCGTGATCCGCGGAAACGAAGTCTCTCAGATCGCTCCTGGACAGACCGGAA

ATATCGCTGACTACAACTACAAGCTGCCAGACGACTTCACTGGCTGCGTGATCGCTTG

GAACTCAAACAAGCTGGACTCCAAGCCTTCTGGCAACTACAACTACCTGTACAGGCT

GTTCAGAAAGTCAAAGCTGAAGCCTTTCGAGCGCGACATCTCAACCGAAATCTACCA

GGCTGGTAATAAGCCCTGCAACGGTGTGGCTGGCCCTAACTGCTACTCTCCCCTGCAG

TCCTACGGTTTCCGTCCAACCTACGGAGTCGGTCATCAGCCTTACCGTGTGGTCGTGC

TGAGCTTCGAACTGCTCCACGCTCCTGCTACTGTGTGCGGTCCCAAGAAGTCTACTA

ACCTGGTCAAAAACAAATGTGTCAACTTCAACTTCAACGGTTAA

SEQ ID NO.14 Complete XBB.1.5 sequence-2 signal peptide-Trx tag-6His tag-EK restriction site-RBD sequence

MLLVNQSHQGFNKEHTSKMVSAIVLYVLLAAAAHSAFAADSIHIKDSDDLKNRLAE

AGDKLVVIDFMATWCGPCKMIGPKLDEMANEMSDCIVVLKVDVDECEDIATEYNINSM

PTFVFVKNSKKIEEFSGANVDKLRNTIIKLKLAGSGSGHMHHHHHHSSGDDDDKVQPTE

SIVRFPNITNLCPFHEVFNATTFASVYAWNRKRISNCVADYSVIYNSASFSAFKCYGVSPT

KLNDLCFTNVYADSFVIRGNEVSQIAPGQTGNIADYNYKLPDDFTGCVIAWNSNKLDSK

PSGNYNYLYRLFRKSKLKPFERDISTEIYQAGNKPCNGVAGPNCYSPLQSYGFRPTYGVG

HQPYRVVVLSFELLHAPATVCGPKKSTNLVKNKCVNFNFNG

SEQ ID NO. 15 - nucleotide sequence encoding SEQ ID NO. 14

ATGCTACTAGTAAATCAGTCACACCAAGGTTTCAACAAGGAACACACCAGCAAG

ATGGTGAGCGCTATCGTGCTGTACGTCCTGCTGGCCGCTGCTGCTCACAGCGCTTTCG

CTGCTGACTCCATCCACATCAAGGACAGCGACGACCTGAAGAACCGTCTGGCCGAG

GCCGGTGACAAGCTGGTCGTCATCGACTTCATGGCCACTTGGTGCGGTCCTTGCAAG

ATGATCGGCCCTAAGCTGGACGAGATGGCTAACGAGATGTCCGACTGCATCGTGGTC

CTGAAGGTGGACGTCGACGAGTGCGAGGACATCGCCACCGAATACAACATCAACAG

CATGCCCACCTTCGTGTTCGTGAAGAACAGCAAGAAGATCGAGGAATTTTCCGGCGC

TAACGTCGACAAGCTGCGTAACACCATCATCAAGCTGAAGCTGGCCGGCTCCGGCTC

CGGCCACATGCATCACCACCACCACCATTCCTCCGGTGACGACGACGACAAGGTGCA

GCCAACCGAATCTATCGTCAGATTCCCAAACATCACTAACCTGTGCCCTTTCCACGAG

GTGTTCAACGCTACCACTTTCGCCAGCGTCTACGCTTGGAACCGCAAGCGTATCAGC

AACTGCGTCGCCGACTACTCTGTGATCTACAACTCCGCTAGCTTCTCTGCTTTCAAGT
GCTACGGCGTGTCACCTACCAAGCTGAACGACCTGTGCTTCACTAACGTCTACGCCG
ACTCCTTCGTGATCCGCGGAAACGAAGTCTCTCAGATC*GCTCCTGGACAGACCGGAAA
T*ATCGCTGACTACAACTACAAGCTGCCAGACGACTTCACTGGCTGCGTGATCGCTTGG
AACTCAAACAAGCTGGACTCC*AAGCCTTCTGGCAACTACAACTACCTG*TACAGGCTGTT
CAGAAAGTCAAAGCTGAAGCCTTTCGAGCGCGACATCTCAACCGAAATCTACCAGGC
TGGTAATAAGCCCTGCAACGGTGTGGCTGGCCCTAACTGCTACTCTCCCCTGCAGTCC
TACGGTTTCCGTCCAACCTACGGAGTCGGTCATCAGCCTTACCGTGTGGTCGTGCTGA
GCTTCGAACTGCTCCACGCTCCTGCTACTGTGTGCGGTCCCAAGAAGTCTACTAACCT
GGTCAAAAACAAATGTGTCAACTTCAACTTCAACGGTTAA

SEQ ID NO.16 Complete XBB.1.5 sequence-3 signal peptide-Trx tag-6His tag-EK restriction site-RBD sequence-HR1 sequence-HR2 sequence

MLLVNQSHQGFNKEHTSKMVSAIVLYVLLAAAAHSAFAADSIHIKDSDDLKNRLAE
AGDKLVVIDFMATWCGPCKMIGPKLDEMANEMSDSIVVLKVDVDECEDIATEYNINSMP
TFVFVKNSKKIEEFSGANVDKLRNTIIKLKLAGSGSGHMHHHHHHSSGDDDDKVQPTES
IVRFPNITNLCPFHEVFNATTFASVYAWNRKRISNCVADYSVIYNFAPFFAFKCYGVSPTK
LNDLCFTNVYADSFVIRGNEVSQIAPGQTGNIADYNYKLPDDFTGCVIAWNSNKLDSKPS
GNYNYLYRLFRKSKLKPFERDISTEIYQAGNKPCNGVAGPNCYSPLQSYGFRPTYGVGH
QPYRVVVLSFELLHAPATVCGPKKSTNLVKNKSVNFNFNGLYENQKLIANQFNSAIGKIQ
DSLSSTASALGKLQDVVNQNAQALNTLVKQLKNHTSPDVDLGDISGINASVVNIQKEID
RLNEVAKNLNESLIDLQEL

SEQ ID NO. 17 - nucleotide sequence encoding SEQ ID NO. 16

ATGCTGCTGGTGAACCAGAGCCACCAGGGTTTCAACAAGGAACACACTAGCAA
GATGGTGTCTGCTATCGTCCTCTACGTGCTGCTCGCCGCCGCTGCTCACTCAGCTTTC
GCCGCTGACTCTATCCACATCAAGGACTCCGACGACCTCAAGAACCGTTTGGCCGAA
GCCGGCGACAAGCTGGTCGTTATCGACTTCATGGCTACCTGGTGTGGCCCTTGCAAGA
TGATCGGACCTAAGCTGGACGAAATGGCTAACGAGATGAGCGACAGCATCGTCGTTC
TGAAGGTGGACGTCGACGAATGTGAGGACATCGCCACTGAGTACAACATCAACAGCA
TGCCTACCTTCGTTTTCGTGAAGAACTCTAAGAAGATCGAGGAGTTCTCTGGTGCCA

ACGTGGACAAGCTCCGTAACACCATCATCAAGCTCAAGCTGGCCGGCAGCGGTAGCG

GTCACATGCACCACCACCACCACCATTCAAGCGGCGACGACGACGACAAGGTGCAG

CCAACCGAATCAATCGTCCGCTTCCCAAACATCACTAACCTGTGCCCATTCCACGAGG

TGTTCAACGCCACCACTTTCGCCTCTGTCTACGCTTGGAACCGCAAGCGCATCTCCAA

CTGTGTCGCCGACTACAGCGTGATATACAACTTCGCTCCTTTCTTCGCATTCAAGTGTT

ACGGAGTGTCCCCTACCAAGCTCAACGATTTGTGCTTCACAAACGTGTACGCTGACA

GCTTCGTGATCCGTGGTAACGAGGTTTCTCAAATCGCCCCTGGTCAGACAGGTAACAT

CGCTGACTACAACTACAAGCTCCCAGACGACTTCACTGGCTGTGTTATCGCTTGGAA

CTCAAACAAGCTGGACTCTAAGCCGTCTGGTAACTACAACTACCTCTACCGTCTGTTC

CGCAAGAGCAAGCTCAAGCCATTCGAACGTGACATCAGCACAGAGATCTACCAAGC

CGGTAACAAGCCCTGCAACGGCGTTGCCGGACCTAACTGCTACTCTCCCCTCCAGTC

CTACGGATTCCGCCCAACCTACGGCGTGGGTCACCAGCCATACCGCGTGGTTGTGCT

GAGCTTCGAACTGCTGCACGCCCCCGCTACCGTGTGTGGCCCTAAGAAGAGCACTAA

CCTCGTTAAGAACAAGTCCGTGAACTTCAACTTCAACGGTCTCTACGAAAACCAGAA

GCTGATCGCTAACCAGTTCAACTCCGCCATCGGCAAGATCCAGGACTCCCTGTCCTCC

ACAGCTTCCGCTCTGGGCAAGCTGCAGGACGTTGTGAACCAAAACGCTCAGGCCCT

GAACACACTGGTGAAGCAACTGAAGAACCACACCAGCCCCGACGTTGACCTGGGTG

ACATCAGCGGTATCAACGCTAGCGTGGTGAACATCCAGAAGGAGATCGACAGGCTGA

ACGAAGTGGCTAAGAACTTGAACGAGAGCCTCATCGACCTGCAGGAACTGTAA

SEQ ID NO.18 Complete XBB.1.5 sequence-4 signal peptides-Trx tag-6His tag-EK restriction site-RBD sequence-HR1 sequence-HR2 sequence

MLLVNQSHQGFNKEHTSKMVSAIVLYVLLAAAAHSAFAADSIHIKDSDDLKNRLAE

AGDKLVVIDFMATWCGPCKMIGPKLDEMANEMSDSIVVLKVDVDECEDIATEYNINSMP

TFVFVKNSKKIEEFSGANVDKLRNTIIKLKLAGSGSGHMHHHHHHSSGDDDDKVQPTES

IVRFPNITNLCPFHEVFNATTFASVYAWNRKRISNCVADYSVIYNSASFSAFKCYGVSPTK

LNDLCFTNVYADSFVIRGNEVSQIAPGQTGNIADYNYKLPDDFTGCVIAWNSNKLDSKPS

GNYNYLYRLFRKSKLKPFERDISTEIYQAGNKPCNGVAGPNCYSPLQSYGFRPTYGVGH

QPYRVVVLSFELLHAPATVCGPKKSTNLVKNKSVNFNFNGLYENQKLIANQFNSAIGKIQ

DSLSSTASALGKLQDVVNQNAQALNTLVKQLKNHTSPDVDLGDISGINASVVNIQKEID

RLNEVAKNLNESLIDLQEL

SEQ ID NO. 19 - nucleotide sequence encoding SEQ ID NO. 18

ATGCTGCTGGTGAACCAGAGCCACCAGGGTTTCAACAAGGAACACACTAGCAA
GATGGTGTCTGCTATCGTCCTCTACGTGCTGCTCGCCGCCGCTGCTCACTCAGCTTTC
GCCGCTGACTCTATCCACATCAAGGACTCCGACGACCTCAAGAACCGTTTGGCCGAA
GCCGGCGACAAGCTGGTCGTTATCGACTTCATGGCTACCTGGTGTGGCCCTTGCAAGA
TGATCGGACCTAAGCTGGACGAAATGGCTAACGAGATGAGCGACAGCATCGTCGTTC
TGAAGGTGGACGTCGACGAATGTGAGGACATCGCCACTGAGTACAACATCAACAGCA
TGCCTACCTTCGTTTTCGTGAAGAACTCTAAGAAGATCGAGGAGTTCTCTGGTGCCA
ACGTGGACAAGCTCCGTAACACCATCATCAAGCTCAAGCTGGCCGGCAGCGGTAGCG
GTCACATGCACCACCACCACCACCATTCAAGCGGCGACGACGACGACAAGGTGCAG
CCAACCGAATCAATCGTCCGCTTCCCAAACATCACTAACCTGTGCCCATTCCACGAGG
TGTTCAACGCCACCACTTTCGCCTCTGTCTACGCTTGGAACCGCAAGCGCATCTCCAA
CTGTGTCGCCGACTACAGCGTGATATACAACTCCGCTTCTTTCTCTGCATTCAAGTGTT
ACGGAGTGTCCCCTACCAAGCTCAACGATTTGTGCTTCACAAACGTGTACGCTGACA
GCTTCGTGATCCGTGGTAACGAGGTTTCTCAAATCGCCCCTGGTCAGACAGGTAACAT
CGCTGACTACAACTACAAGCTCCCAGACGACTTCACTGGCTGTGTTATCGCTTGGAA
CTCAAACAAGCTGGACTCTAAGCCGTCTGGTAACTACAACTACCTCTACCGTCTGTTC
CGCAAGAGCAAGCTCAAGCCATTCGAACGTGACATCAGCACAGAGATCTACCAAGC
CGGTAACAAGCCCTGCAACGGCGTTGCCGGACCTAACTGCTACTCTCCCCTCCAGTC
CTACGGATTCCGCCCAACCTACGGCGTGGGTCACCAGCCATACCGCGTGGTTGTGCT
GAGCTTCGAACTGCTGCACGCCCCCGCTACCGTGTGTGGCCCTAAGAAGAGCACTAA
CCTCGTTAAGAACAAGTCCGTGAACTTCAACTTCAACGGTCTCTACGAAAACCAGAA
GCTGATCGCTAACCAGTTCAACTCCGCCATCGGCAAGATCCAGGACTCCCTGTCCTCC
ACAGCTTCCGCTCTGGGCAAGCTGCAGGACGTTGTGAACCAAAACGCTCAGGCCCT
GAACACACTGGTGAAGCAACTGAAGAACCACACCAGCCCCGACGTTGACCTGGGTG
ACATCAGCGGTATCAACGCTAGCGTGGTGAACATCCAGAAGGAGATCGACAGGCTGA
ACGAAGTGGCTAAGAACTTGAACGAGAGCCTCATCGACCTGCAGGAACTGTAA

SEQ ID NO.20 Complete S-XBB.1.5 amino acid sequence, Ad5$_{XBB.1.5}$ adenovirus vaccine antigen amino acid sequence

MFVFLVLLPLVSSQCVNLITRTQSYTNSFTRGVYYPDKVFRSSVLHSTQDLFLPFFSN

VTWFHAIHVSGTNGTKRFDNPALPFNDGVYFASTEKSNIIRGWIFGTTLDSKTQSLLIVNN

ATNVVIKVCEFQFCNDPFLDVYQKNNKSWMESEFRVYSSANNCTFEYVSQPFLMDLEG

KEGNFKNLREFVFKNIDGYFKIYSKHTPINLERDLPQGFSALEPLVDLPIGINITRFQTLLA

LHRSYLTPVDSSSGWTAGAAAYYVGYLQPRTFLLKYNENGTITDAVDCALDPLSETKCT

LKSFTVEKGIYQTSNFRVQPTESIVRFPNITNLCPFHEVFNATTFASVYAWNRKRISNCVAD

YSVIYNFAPFFAFKCYGVSPTKLNDLCFTNVYADSFVIRGNEVSQIAPGQTGNIADYNYK

LPDDFTGCVIAWNSNKLDSKPSGNYNYLYRLFRKSKLKPFERDISTEIYQAGNKPCNGVA

GPNCYSPLQSYGFRPTYGVGHQPYRVVVLSFELLHAPATVCGPKKSTNLVKNKCVNFNF

NGLTGTGVLTESNKKFLPFQQFGRDIADTTDAVRDPQTLEILDITPCSFGGVSVITPGTNTS

NQVAVLYQGVNCTEVPVAIHADQLTPTWRVYSTGSNVFQTRAGCLIGAEYVNNSYECDI

PIGAGICASYQTQTKSHRRARSVASQSIIAYTMSLGAENSVAYSNNSIAIPTNFTISVTTEIL

PVSMTKTSVDCTMYICGDSTECSNLLLQYGSFCTQLKRALTGIAVEQDKNTQEVFAQVK

QIYKTPPIKYFGGFNFSQILPDPSKPSKRSFIEDLLFNKVTLADAGFIKQYGDCLGDIAARD

LICAQKFNGLTVLPPLLTDEMIAQYTSALLAGTITSGWTFGAGAALQIPFAMQMAYRFNG

IGVTQNVLYENQKLIANQFNSAIGKIQDSLSSTASALGKLQDVVNHNAQALNTLVKQLSS

KFGAISSVLNDILSRLDKVEAEVQIDRLITGRLQSLQTYVTQQLIRAAEIRASANLAATKM

SECVLGQSKRVDFCGKGYHLMSFPQSAPHGVVFLHVTYVPAQEKNFTTAPAICHDGKAH

FPREGVFVSNGTHWFVTQRNFYEPQIITTDNTFVSGNCDVVIGIVNNTVYDPLQPELDSF

KEELDKYFKNHTSPDVDLGDISGINASVVNIQKEIDRLNEVAKNLNESLIDLQELGKYEQ

YIKWPWYIWLGFIAGLIAIVMVTIMLCCMTSCCSCLKGCCSCGSCCKFDEDDSEPVLKG

VKLHYT

SEQ ID NO.21 Nucleotide sequence encoding SEQ ID NO.20, $Ad5_{XBB.1.5}$ adenovirus vaccine antigen nucleotide sequence

ATGTTCGTGTTCCTGGTGCTGCTGCCCCTGGTGAGCAGCCAATGCGTGAACCTGA

TCACAAGAACACAGAGCTACACCAACAGCTTCACAAGAGGCGTGTACTACCCCGAC

AAGGTGTTCAGAAGCAGCGTGCTGCATTCCACCCAAGACCTGTTCCTCCCCTTCTTTT

CCAACGTGACCTGGTTCCACGCCATCCACGTGAGCGGCACCAACGGCACCAAGAGA

TTCGACAACCCCGCCCTGCCCTTCAACGACGGCGTGTACTTCGCTAGCACCGAGAAG

AGCAACATCATCAGAGGCTGGATCTTCGGCACCACCCTGGACAGCAAGACACAGAG

CCTGCTGATCGTGAACAATGCCACCAACGTGGTGATCAAGGTGTGCGAGTTTCAGTT

CTGCAACGACCCCTTCCTGGACGTGTATCAGAAGAACAACAAGAGCTGGATGGAGA

GCGAGTTCAGAGTGTACAGCAGCGCTAACAACTGCACCTTCGAGTACGTGAGCCAAC

CCTTCCTGATGGACCTGGAGGGCAAGGAGGGCAACTTCAAGAACCTCAGAGAGTTC

GTGTTCAAGAACATCGACGGCTACTTCAAGATCTACAGCAAGCACACCCCCATCAAC

CTGGAGAGAGACCTGCCCCAAGGCTTCAGCGCCCTGGAGCCCCTGGTGGACCTGCC

CATCGGCATCAACATCACAAGATTTCAGACCCTGCTGGCCCTGCACAGAAGCTATCTG

ACACCTGTGGACAGCAGCAGCGGCTGGACAGCTGGGGCCGCTGCCTACTACGTGGG

CTACCTGCAGCCTAGAACCTTCCTGCTGAAGTACAACGAGAACGGCACAATCACCGA

TGCCGTGGATTGCGCCCTGGACCCCCTGAGCGAGACCAAGTGCACCCTGAAGAGCTT

CACCGTGGAGAAGGGCATCTATCAGACAAGCAACTTCAGAGTGCAGCCCACCGAGA

GCATCGTGAGATTCCCCAACATCACCAACCTGTGCCCCTTCCACGAGGTGTTCAACG

CCACCACCTTCGCTAGCGTGTACGCTTGGAACAGAAAAAGAATCAGCAACTGCGTGG

CCGACTACAGCGTGATCTACAACTTCGCCCCCTTCTTCGCCTTCAAGTGCTACGGGGT

GAGCCCCACCAAGCTGAACGACCTGTGCTTCACCAACGTGTACGCCGACAGCTTCGT

GATCAGAGGCAACGAGGTGAGCCAAATCGCCCCTGGGCAGACCGGCAACATCGCCG

ACTACAACTACAAGCTGCCCGACGACTTCACCGGCTGCGTGATCGCCTGGAACAGCA

ACAAGCTGGACAGCAAACCTAGCGGCAACTACAACTACCTGTACAGACTGTTCAGAA

AGAGCAAGCTGAAGCCCTTCGAGAGAGACATCAGCACCGAGATCTACCAAGCCGGC

AACAAGCCCTGCAACGGCGTGGCCGGCCCCAACTGCTACAGCCCCCTGCAGAGCTA

CGGCTTCAGACCCACCTACGGCGTGGGCCATCAGCCCTACAGAGTGGTCGTGCTGAG

CTTCGAGCTGCTGCACGCCCCCGCCACCGTGTGCGGCCCCAAGAAGAGCACCAACC

TGGTGAAGAACAAGTGCGTGAACTTCAACTTCAACGGCCTCACCGGCACCGGCGTG

CTGACCGAGAGCAACAAGAAGTTCCTGCCCTTTCAGCAGTTCGGCAGAGACATCGCC

GACACCACCGACGCCGTGAGAGACCCTCAGACCCTGGAGATCCTGGACATCACCCCC

TGTAGCTTCGGCGGCGTGAGCGTGATCACCCCCGGCACCAACACAAGCAACCAAGT

GGCCGTGCTGTACCAAGGCGTGAACTGCACCGAGGTGCCCGTGGCCATCCACGCCGA

TCAGCTGACCCCCACCTGGAGAGTCTACTCCACCGGCAGCAACGTGTTTCAGACAAG

AGCCGGCTGCCTGATCGGCGCCGAGTACGTGAACAACAGCTACGAGTGCGACATCCC

CATCGGCGCCGGCATCTGCGCTAGCTATCAGACACAGACCAAGAGCCACGGCAGCGC

TAGCAGCGTGGCTAGCCAAAGCATCATCGCCTACACCATGAGCCTGGGCGCCGAGAA

CAGCGTGGCCTACAGCAACAACAGCATCGCCATCCCCACCAACTTCACCATCAGCGT

GACCACCGAGATCCTGCCCGTCAGCATGACCAAGACAAGCGTGGACTGCACCATGTA

CATCTGCGGCGACAGCACCGAGTGCAGCAACCTGCTCCTGCAGTACGGCAGCTTCTG

CACACAGCTGAAGAGAGCCCTGACCGGCATCGCCGTGGAGCAAGACAAGAACACCC

AAGAGGTGTTCGCCCAAGTGAAGCAGATCTACAAGACCCCCCCCATCAAGTACTTCG

GCGGCTTCAACTTCAGCCAAATCCTCCCCGACCCCTCCAAACCTAGCAAGAGAAGCT

TCATCGAGGACCTGCTGTTCAACAAGGTGACCCTGGCCGACGCCGGCTTCATCAAGC

AGTACGGCGACTGCCTGGGCGATATCGCCGCTAGAGACCTGATCTGCGCTCAGAAGT

TCAATGGCCTGACCGTGCTGCCCCCCCTGCTGACCGACGAGATGATCGCTCAGTATAC

AAGCGCTCTGCTCGCTGGCACCATTACAAGCGGGTGGACCTTCGGCGCTGGCGCTGC

CCTGCAGATCCCCTTCGCCATGCAGATGGCCTACAGATTCAACGGCATCGGCGTGACA

CAGAACGTGCTGTACGAGAATCAGAAGCTGATCGCCAATCAGTTCAACAGCGCCATC

GGCAAGATCCAAGACAGCCTGAGCAGCACCGCTAGCGCCCTGGGCAAGCTGCAAGA

CGTGGTGAACCACAACGCCCAAGCCCTGAACACCCTGGTGAAGCAGCTGAGCAGCA

AGTTCGGCGCCATCAGCAGCGTGCTCAACGACATCCTGAGCAGACTGGACCCCCCCG

AGGCCGAGGTGCAGATCGATAGACTGATCACCGGCAGACTGCAGAGCCTGCAGACCT

ACGTGACACAGCAGCTGATCAGAGCCGCCGAGATCAGAGCTAGCGCCAACCTGGCC

GCCACCAAGATGAGCGAGTGCGTGCTGGGGCAGAGCAAGAGAGTGGACTTCTGCGG

CAAGGGCTACCACCTGATGAGCTTCCCTCAGAGCGCCCCCCACGGCGTGGTGTTCCT

GCACGTGACCTACGTGCCCGCCCAAGAGAAGAACTTCACCACAGCCCCCGCCATCTG

CCACGACGGCAAGGCCCACTTCCCTAGAGAGGGCGTGTTCGTGAGCAACGGCACCC

ACTGGTTCGTGACACAGAGAAACTTCTACGAGCCTCAGATCATCACCACCGACAACA

CCTTCGTGAGCGGCAACTGCGACGTGGTGATCGGCATCGTGAATAATACCGTGTACG

ACCCCCTGCAGCCCGAGCTGGACAGCTTCAAGGAGGAGCTGGACAAGTATTTCAAG

AACCATACAAGCCCCGACGTGGACCTGGGCGACATCAGCGGCATCAACGCTAGCGTG

GTGAACATTCAGAAGGAAATCGACAGACTCAACGAGGTGGCCAAGAACCTGAACGA

GAGCCTGATCGACCTGCAAGAGCTGGGCAAGTACGAGCAGTACATCAAGTGGCCCTG

GTACATCTGGCTGGGCTTCATCGCCGGCCTGATCGCCATCGTGATGGTGACCATCATG

CTGTGCTGCATGACAAGCTGCTGCAGCTGCCTGAAGGGCTGTTGCAGCTGCGGCAGC

TGCTGCAAGTTCGACGAGGACGACAGCGAGCCCGTGCTGAAGGGCGTGAAGCTGCA

CTACACCTGA

The applicant found that the full-length gene of the S protein of the Omicron variant BA.5, as shown in SEQ ID NO.23, was used to prepare an adenovirus vaccine, and then the RBD sequence or RBD-HR in the S protein of the Omicron variant BA.5 and XBB.1.5 was used to prepare a recombinant protein vaccine, and the combination of adenovirus vaccine and recombinant protein vaccine also had a good preventive and therapeutic effect on cross-infection caused by SARS-CoV-2 or its mutant viruses.

SEQ ID NO.22 Complete S-$_{BA.5}$ amino acid sequence, antigen amino acid sequence of Ad5$_{BA.5}$ adenovirus vaccine

MFVFLVLLPLVSSQCVNLITRTQSYTNSFTRGVYYPDKVFRSSVLHSTQDLFLPFFSN

VTWFHAISGTNGTKRFDNPVLPFNDGVYFASTEKSNIIRGWIFGTTLDSKTQSLLIVNNAT

NVVIKVCEFQFCNDPFLDVYYHKNNKSWMESEFRVYSSANNCTFEYVSQPFLMDLEGK

QGNFKNLREFVFKNIDGYFKIYSKHTPINLGRDLPQGFSALEPLVDLPIGINITRFQTLLAL

HRSYLTPGDSSSGWTAGAAAYYVGYLQPRTFLLKYNENGTITDAVDCALDPLSETKCTL

KSFTVEKGIYQTSNFRVQPTESIVRFPNITNLCPFDEVFNATRFASVYAWNRKRISNCVAD

YSVLYNFAPFFAFKCYGVSPTKLNDLCFTNVYADSFVIRGNEVSQIAPGQTGNIADYNYK

LPDDFTGCVIAWNSNKLDSKVGGNYNYRYRLFRKSNLKPFERDISTEIYQAGNKPCNGV

AGVNCYFPLQSYGFRPTYGVGHQPYRVVVLSFELLHAPATVCGPKKSTNLVKNKCVNFN

FNGLTGTGVLTESNKKFLPFQQFGRDIADTTDAVRDPQTLEILDITPCSFGGVSVITPGTNT

SNQVAVLYQGVNCTEVPVAIHADQLTPTWRVYSTGSNVFQTRAGCLIGAEYVNNSYECD

IPIGAGICASYQTQTKSHRRARSVASQSIIAYTMSLGAENSVAYSNNSIAIPTNFTISVTTEIL

PVSMTKTSVDCTMYICGDSTECSNLLLQYGSFCTQLKRALTGIAVEQDKNTQEVFAQVK

QIYKTPPIKYFGGFNFSQILPDPSKPSKRSFIEDLLFNKVTLADAGFIKQYGDCLGDIAARD

LICAQKFNGLTVLPPLLTDEMIAQYTSALLAGTITSGWTFGAGAALQIPFAMQMAYRFNG

IGVTQNVLYENQKLIANQFNSAIGKIQDSLSSTASALGKLQDVVNHNAQALNTLVKQLSS

KFGAISSVLNDILSRLDPPEAEVQIDRLITGRLQSLQTYVTQQLIRAAEIRASANLAATKM

SECVLGQSKRVDFCGKGYHLMSFPQSAPHGVVFLHVTYVPAQEKNFTTAPAICHDGKAH

FPREGVFVSNGTHWFVTQRNFYEPQIITTDNTFVSGNCDVVIGIVNNTVYDPLQPELDSF

KEELDKYFKNHTSPDVDLGDISGINASVVNIQKEIDRLNEVAKNLNESLIDLQELGKYEQ

YIKWPWYIWLGFIAGLIAIVMVTIMLCCMTSCCSCLKGCCSCGSCCKFDEDDSEPVLKG

VKLHYT

SEQ ID NO.23 Nucleotide sequence encoding SEQ ID NO.22, antigen nucleotide sequence of Ad5$_{BA.5}$ adenovirus vaccine

ATGTTCGTGTTCCTGGTGCTGCTGCCCCTGGTGAGCAGCCAATGCGTGAACCTGA
TCACAAGAACACAGAGCTACACCAACAGCTTCACAAGAGGCGTGTACTACCCCGAC
AAGGTGTTCAGAAGCAGCGTCCTGCATTCCACCCAAGACCTGTTTCTCCCCTTCTTCA
GCAACGTGACCTGGTTCCACGCCATCAGCGGCACCAACGGCACCAAGAGATTCGAC
AACCCCGTGCTGCCCTTCAACGACGGCGTGTACTTCGCTAGCACCGAGAAGAGCAAC
ATCATCAGAGGCTGGATCTTCGGCACCACCCTGGACAGCAAGACACAGAGCCTGCTG
ATCGTGAACAACGCCACAAACGTGGTGATCAAGGTGTGCGAGTTTCAGTTCTGCAAC
GACCCCTTCCTGGACGTGTACTACCACAAGAACAACAAGAGCTGGATGGAGAGCGA
GTTCCGGGTGTACAGCAGCGCCAACAACTGCACCTTCGAGTACGTGAGCCAACCCTT
CCTGATGGACCTGGAGGGCAAGCAAGGCAACTTCAAGAACCTGAGAGAGTTCGTGT
TCAAGAACATCGACGGCTACTTCAAGATCTACAGCAAGCACACCCCCATCAACCTGG
GCAGAGACCTGCCCCAAGGCTTCAGCGCCCTGGAGCCCCTGGTGGACCTGCCCATCG
GCATCAACATCACAAGATTTCAGACACTGCTCGCCCTGCACCGGAGCTACCTCACCC
CTGGCGACAGCAGCTCCGGCTGGACCGCTGGGGCTGCCGCCTACTACGTGGGCTACC
TGCAGCCTAGAACCTTCCTGCTGAAGTACAACGAGAACGGCACCATCACCGACGCTG
TGGACTGTGCCCTGGACCCCCTGAGCGAGACCAAGTGCACCCTGAAGAGCTTCACC
GTGGAGAAGGGCATCTATCAGACAAGCAACTTCAGAGTGCAGCCCACCGAGAGCAT
CGTGAGATTCCCCAACATCACCAACCTGTGCCCCTTCGACGAGGTGTTCAACGCCAC
AAGATTCGCTAGCGTGTACGCCTGGAACAGAAAGAGAATCAGCAACTGCGTGGCCG
ACTACAGCGTGCTGTACAACTTCGCCCCCTTCTTCGCCTTCAAATGCTACGGCGTCAG
CCCCACCAAGCTGAACGACCTGTGCTTCACCAACGTGTACGCCGACAGCTTCGTGAT
CAGAGGCAACGAGGTGAGCCAAATCGCCCCCGGGCAGACCGGCAACATCGCCGACT
ATAACTACAAACTGCCCGACGACTTCACCGGCTGCGTGATCGCCTGGAACTCCAACA
AGCTGGACAGCAAAGTGGGCGGCAACTACAACTACAGATACAGACTGTTCAGAAAG
AGCAACCTGAAGCCCTTCGAGAGAGACATCAGCACCGAGATCTACCAAGCCGGCAA
CAAGCCCTGCAACGGCGTGGCCGGCGTGAACTGCTACTTCCCCCTGCAGAGCTACGG
CTTCAGACCCACCTACGGCGTGGGCCATCAGCCCTACAGAGTGGTCGTGCTGAGCTT
CGAGCTGCTGCACGCCCCTGCCACAGTGTGCGGCCCCAAGAAGAGCACCAACCTGG
TGAAGAACAAGTGCGTGAACTTCAACTTCAACGGCCTGACCGGCACCGGCGTGCTG
ACCGAGAGCAACAAGAAGTTCCTGCCCTTTCAGCAGTTCGGCAGAGACATCGCCGA
CACCACCGATGCTGTGAGAGACCCTCAGACCCTGGAGATCCTGGACATCACCCCTTG
CAGCTTCGGCGGCGTGAGCGTGATCACCCCCGGCACCAACACAAGCAACCAAGTGG

CCGTGCTGTACCAAGGCGTCAACTGCACAGAGGTGCCCGTGGCCATCCACGCCGATC

AGCTGACCCCCACCTGGAGAGTGTACTCCACCGGCAGCAACGTGTTTCAGACAAGA

GCCGGCTGCCTGATCGGCGCCGAGTACGTGAACAACAGCTACGAGTGCGACATCCCC

ATCGGCGCCGGCATCTGCGCTAGCTATCAGACACAGACCAAGAGCCACAGAAGAGCT

AGAAGCGTGGCTAGCCAAAGCATCATCGCCTACACCATGAGCCTGGGCGCCGAGAAC

AGCGTGGCCTACAGCAACAACAGCATCGCCATCCCCACCAACTTCACCATCAGCGTG

ACCACCGAGATCCTGCCTGTGAGCATGACCAAGACAAGCGTGGACTGCACCATGTAC

ATCTGCGGCGACAGCACCGAGTGCAGCAACCTGCTCCTGCAGTACGGCAGCTTCTGC

ACACAGCTGAAGAGAGCCCTGACCGGCATCGCCGTGGAGCAAGACAAGAACACCCA

AGAGGTGTTCGCCCAAGTGAAGCAGATCTACAAGACCCCCCCCATCAAGTACTTCGG

CGGCTTCAACTTCAGCCAAATCCTGCCCGACCCTAGCAAGCCTAGCAAGCGGAGCTT

CATCGAGGACCTGCTGTTCAACAAGGTGACCCTGGCCGACGCCGGCTTCATCAAGCA

GTACGGCGACTGCCTCGGCGACATCGCTGCTAGAGACCTGATCTGCGCTCAGAAGTT

CAACGGCCTCACAGTGCTGCCCCCCCTGCTGACCGACGAGATGATCGCTCAGTACAC

CTCCGCTCTGCTGGCCGGCACAATTACATCCGGCTGGACCTTCGGCGCCGGCGCCGC

CCTGCAAATCCCCTTCGCCATGCAGATGGCCTACAGATTCAACGGCATCGGCGTGACA

CAGAACGTGCTGTACGAGAATCAGAAGCTGATCGCCAATCAGTTCAACAGCGCCATC

GGCAAGATCCAAGACAGCCTGAGCAGCACCGCTAGCGCCCTGGGCAAGCTGCAAGA

CGTGGTGAACCACAACGCCCAAGCCCTGAACACCCTGGTGAAGCAGCTGAGCAGCA

AGTTCGGCGCCATCTCCTCCGTCCTGAACGACATCCTGAGCAGACTGGACCCCCCCG

AGGCCGAGGTGCAGATTGACAGACTGATTACCGGCAGACTGCAGAGCCTGCAGACCT

ACGTGACACAGCAGCTGATCAGAGCCGCCGAGATCAGAGCTAGCGCCAACCTGGCC

GCCACCAAGATGAGCGAGTGCGTGCTGGGGCAGAGCAAGAGAGTGGACTTCTGCGG

CAAGGGCTACCACCTGATGAGCTTCCCTCAGAGCGCCCCCCACGGCGTGGTGTTCCT

GCACGTGACCTACGTGCCCGCCCAAGAGAAGAACTTCACCACCGCTCCCGCCATCTG

CCACGACGGCAAGGCCCACTTCCCTAGAGAGGGCGTGTTCGTGAGCAACGGCACCC

ACTGGTTCGTGACACAGAGAAACTTCTACGAGCCTCAGATCATCACCACCGACAACA

CCTTCGTGAGCGGCAACTGCGACGTGGTGATCGGCATCGTCAACAACACCGTGTACG

ACCCCCTGCAACCCGAGCTGGACAGCTTCAAGGAGGAGCTGGACAAGTACTTTAAG

AACCACACAAGCCCCGACGTGGACCTGGGGGACATCTCCGGCATCAACGCTAGCGT

GGTGAACATTCAGAAGGAAATTGACAGACTGAATGAGGTGGCCAAGAACCTGAACG

AGAGCCTGATCGACCTGCAAGAGCTGGGCAAGTACGAGCAGTACATCAAGTGGCCCT

GGTACATCTGGCTGGGCTTCATCGCCGGCCTGATCGCCATCGTGATGGTGACCATCAT

GCTGTGCTGCATGACAAGCTGCTGCAGCTGTCTGAAGGGCTGCTGCTCCTGCGGCAG

CTGCTGCAAGTTCGACGAGGACGACAGCGAGCCCGTGCTGAAGGGCGTGAAGCTGC

ACTACACCTGA

Beneficial Effects: The present invention has been firstly used for preparing a recombinant protein vaccine against SARS-CoV-2 and its variants, and the main idea of constructing the recombinant protein sequence is based on the RBD sequence in the S protein of SARS-CoV-2 Omicron variants BA.5 and XBB.1.5, and the recombinant sequence formed by the RBD sequence and the heptapeptide repeat regions HR1 and HR2 of the S protein of SARS-CoV-2 for protein construction. Simultaneously, after adding the corresponding vaccine adjuvant, it can better help the host resist the cross infection caused by the Omicron variant XBB and subvariants thereof, which is of great significance for the development of recombinant protein vaccines against SARS-CoV-2 Omicron variant XBB and its subvariants.

Simultaneously, the present invention has been also used for constructing a recombinant adenovirus vector based on the nucleotide sequence of the full-length S protein of SARS-CoV-2 Omicron variants BA.5 and XBB.1.5 to obtain an adenovirus vector vaccine then two or three of the prepared different recombinant adenovirus vector vaccines are combined with different recombinant protein vaccines to obtain a vaccine composition with better prevention or treatment effect against SARS-CoV-2 and its variants, and the preparation of the nasal spray reagent is more conducive to resisting SARS-CoV-2 and its variants.

## BRIEF DESCRIPTION OF THE DRAWINGS

FIG. 1 is a design graph of pFastBac1-GP67-Trx-His-EK-S-RBD(Omicron_XBB.1.5)-HR in Embodiment 1;

FIG. 2 is a 1% agarose gel electrophoresis graph of 1-3# PCR products in Embodiment 1;

FIG. 3 is a PCR-identified 1% agarose gel electrophoresis graph of recombinant bacmid in Embodiment 1;

FIG. 4 is a packaging and amplification flowchart of a recombinant baculovirus in Embodiment 1;

FIG. 5 is a WB verification result graph during baculovirus expansion in Embodiment 1;

FIG. 6 is a result graph of eluting supernatant after baculovirus infection through a Ni-affinity chromatography in Embodiment 1;

FIG. 7 is a result graph of verifying an enzyme cutting effect by an EK restriction enzyme cutting sample in Embodiment 1;

FIG. 8 is a graph of packing purification and SDS-PAGE identification through a Ni-affinity chromatography after EK restriction enzyme cutting in Embodiment 1;

FIG. 9 is the expression identification of XBB.1.5 adenovirus in Embodiment 2;

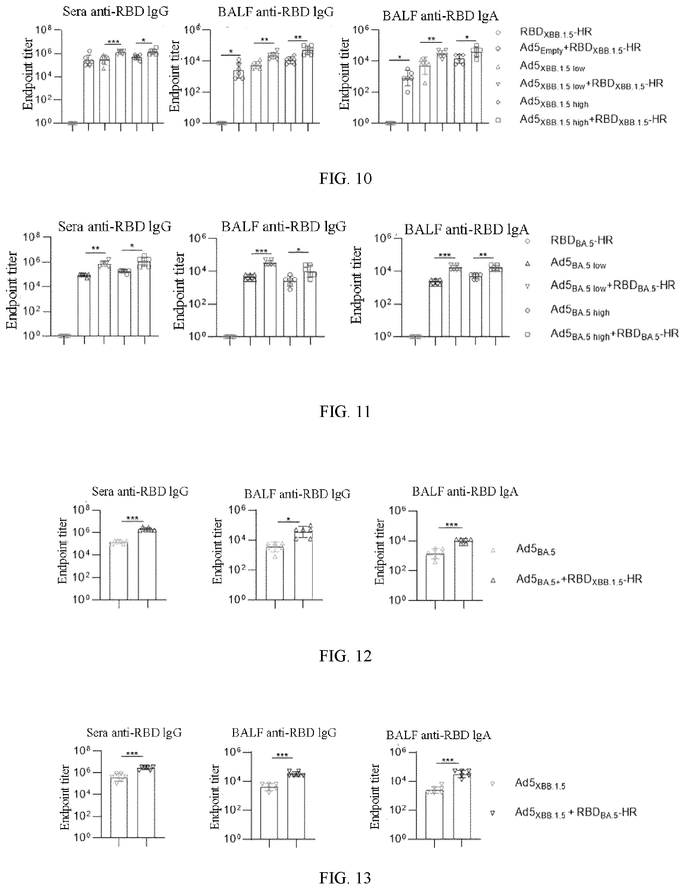

FIG. 10 shows serum IgG and lavage fluid IgG/IgA of the $Ad5_{XBB.1.5}+RBD_{XBB.1.5}$-HR bivalent vaccine in Embodiment 4;

FIG. 11 shows the serum IgG and lavage fluid IgG\IgA of $Ad5_{BA.5}+RBD_{BA.5}$-HR bivalent vaccine in Embodiment 4;

FIG. 12 shows the serum IgG and lavage fluid IgG\IgA of $Ad5_{BA.5}+RBD_{XBB.1.5}$-HR bivalent vaccine in Embodiment 4;

FIG. 13 shows the serum IgG and lavage fluid IgG\IgA of $Ad5_{XBB.1.5}+RBD_{BA.5}$-HR bivalent vaccine in Embodiment 4;

FIG. 14 shows the serum IgG and lavage fluid IgG/IgA data for the trivalent vaccine formulation $Ad5_{XBB.1.5}$ + F-BD$_{XBB.1.5}$-HR + $RBD_{BA.5}$-HR from Embodiment 4;

FIG. 15 shows the neutralizing antibodies in mouse serum of $RBD_{XBB.1.5}$ vaccine (aluminum adjuvant) in Embodiment 5;

FIG. 16 shows the neutralizing antibodies in mouse serum of $RBD_{XBB.1.5}$-HR vaccine (MF59 adjuvant) in Embodiment 5;

FIG. 17 shows the neutralizing antibodies in mouse serum of $RBD_{XBB.1.5}$ vaccine in Embodiment 5;

FIG. 18 shows the serum neutralizing antibody data for the bivalent vaccine $Ad5_{XBB.1.5}$ + $RBD_{XBB.1.5}$-HR from Embodiment 5;

FIG. 19 shows the $Ad5_{XBB.1.5}+RBD_{XBB.1.5}$-HR bivalent vaccine bronchoalveolar lavage fluid neutralizing antibodies in Embodiment 5;

FIG. 20 shows the $Ad5_{XBB.1.5}+RBD_{XBB.1.5}$-HR+$RBD_{BA.5}$-HR trivalent vaccine serum neutralizing antibodies in Embodiment 5;

FIG. 21 shows the $Ad5_{XBB.1.5}+RBD_{XBB.1.5}$-HR bivalent vaccine serum true virus neutralizing antibodies in Embodiment 6;

FIG. 22 shows the mouse immune attack timetable in Embodiment 7;

FIG. 23 shows the virus load of mouse throat swabs in Embodiment 7;

FIG. 24 shows the viral gene/subgene RNA in Embodiment 7;

FIG. 25 shows the histopathological changes of mouse lung tissue in Embodiment 7;

FIG. 26 shows the histopathological scores of mouse lung tissue in Embodiment 7.

## DESCRIPTION OF THE EMBODIMENTS

Terms and abbreviations:

Monophosphate lipid A (MPL); squalene-based oil-in-water emulsion (MF59), recombinant cholera toxin (rCTB), astragalus polysaccharide (APS), phosphatidylethanolamine (PE), phosphatidylcholine (PC), cholesterol (Chol), dioleoyl phosphatidylethanolamine (DOPE)(2,3-dioleoxypropyl) trimethyl ammonium chloride (DOTAP), N-[1-(2,3-dioleyloxy) propyl]-N,N,N-trimethylammonium chloride (DOTMA), cationic cholesterol (DC-Chol), dimethyl trifluoroacetate-2,3-dioleoxypropyl-2-(2-spermamido) ethylammonium (DOSPA), trimethyl dodecyl ammonium bromide (DTAB), trimethyl tetradecyl ammonium bromide (TTAB), trimethyl cetylammonium bromide (CTAB), dimethyl didoctadecyl ammonium bromide (DDAB), CpG ODN (synthetic CpG with unmethylated cytosine and guanine dinucleotide as core sequence).

The present invention first designs a recombinant protein vaccine against SARS-CoV-2 Omicron variant XBB and its subvariants, based on the amino acid sequence at positions 320-545 of the spike protein from SARS-CoV-2 Omicron variants BA.5 and XBB.1.5, in combination with heptad repeat region 1 (HR1) and heptad repeat region 2 (HR2).

The present invention further designs a recombinant adenovirus vaccine against SARS-CoV-2 Omicron variant XBB and its thereof, based on the nucleotide sequences encoding the full-length spike protein of SARS-CoV-2 Omicron BA.5 and XBB.1.5 variants

The present invention also combines different recombinant protein vaccines and different recombinant adenovirus vaccines to prepare bivalent and trivalent nasal spray preparations, which target the S protein of the SARS-CoV-2 virus ,especially block the ACE2 receptor binding region of the S protein, induce the production of antibodies and other immune responses in the body, block the binding of the S protein of SARS-CoV-2 to the ACE2 receptor of the host cell, thereby helping the host resist coronavirus infection, especially having a good preventive and therapeutic effect on cross-infection caused by SARS-CoV-2 or its mutant viruses, such as cross-infection caused by SARS-CoV-2 Omicron variants and their subvariants XBB.1.5, etc..

The solution of the present invention will be explained with reference to embodiments. It will be understood by those skilled in the art that the following embodiments are merely intended for illustrating the present invention, instead of limiting the scope of the present invention. If specific technologies or conditions are not specified in the embodiments, technologies or conditions described in literatures in the art or product specifications should prevail. If manufacturers are not indicated in reagents or instruments used, they are all conventional products that can be obtained through market purchase.

**Embodiment 1 Preparation of recombinant protein by expression using insect baculovirus system (taking S-RBD $_{XBB.1.5}$-HR as an example)**

1. Construction design of S-RBD$_{XBB.1.5}$-HR

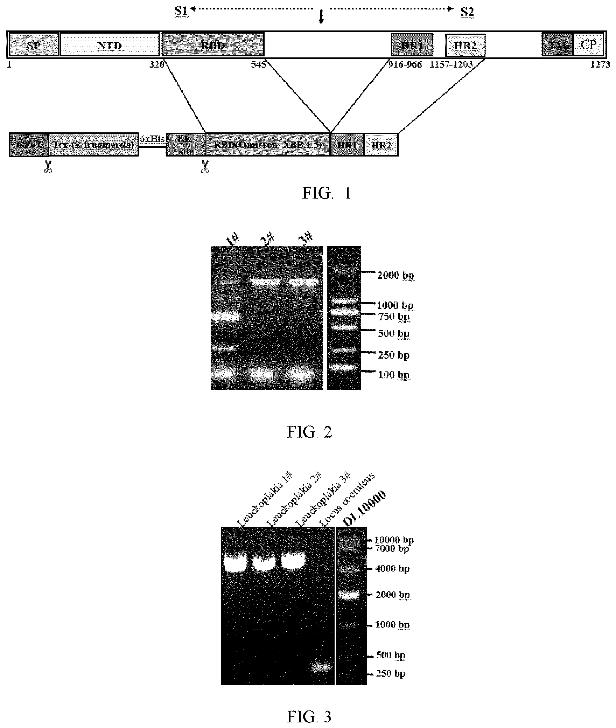

The S protein of SARS-CoV-2 was a membrane-located protein, therefore, in order to mimic its secretion process, the signal peptide sequence of GP67 was added to the N-terminal of the protein during the construction of S-RBD(Omicron_XBB.1.5)-HR protein expression in SARS-CoV-2 to assist in the secretion and expression of the protein, and this signal peptide would be spontaneously removed by insect cells in a protein secretion process; meanwhile, thioredoxin (Trx) tag of Spodoptera frugiperda (S. frugiperda) was added after GP67 signal peptide to assist in S-RBD (Omicron_XBB.1.5)-HR folding, 6xhis tag was added to help subsequent purification, and an EK restriction enzyme cutting site was added to remove both Trx and 6xhis tags. The construction and design of protein expression would be able to remove all non-S-RBD (Omicron_XBB.1.5)-HR redundant amino acids through EK restriction enzyme cutting. The pattern of the expression construction design is shown in FIG. 1.

The amino acid sequence of GP67-Trx-His-EK-S-RBD(Omicron_XBB.1.5) was shown in SEQ ID NO.12, the amino acid sequence of GP67-Trx-His-EK-S-RBD (Omicron_XBB.1.5)-HR was shown in SEQ ID NO.16, and the nucleotide sequences encoding SEQ ID NO.12 and SEQ ID NO.16 were shown in SEQ ID NO.13 and SEQ ID NO.17 respectively.

Similarly, the amino acid sequence of GP67-Trx-His-EK-S-RBD(Omicron_XBB.BA.5)-HR was shown in SEQ ID NO.8, and the nucleotide sequence encoding its amino acid sequence was shown in SEQ ID NO.9.

The above sequences were constructed based on the RBD sequence of S protein 320-545, in which the 52$^{nd}$, 54$^{th}$ and 56$^{th}$ amino acids were F, P and F respectively.

2. Identification for construction of recombinant plasmid

Taking SEQ ID NO.16 as the coding fragment, namely XBB.1.5-3 sequence, the designed coding fragment was cloned into the pFastBac1 vector plasmid and identified by bacterial liquid PCR. The identification of the bacteria solution PCR showed that in the three selected clones, 2# and 3# clones expanded GP67-Trx-His-EK-S-RBD(Omicron_XBB.1.5)-HR fragments successfully, as shown in FIG. 2.

3. Identification of recombinant bacmid

The correctly identified pFastBac1-GP67-Trx-His-EK-S-RBD(Omicron_XBB.1.5)-HR recombinant clone was selected, after extracting the recombinant plasmid, the DH10B competent cell was transformed, and identification of blue-white-spot bacteria solution PCR was performed. The bacteria solution PCR product was detected by 1% agarose gel electrophoresis, and the identification result was as shown in FIG. 3. The white spot showed the bacmid clone that had been recombined, and the blue spot showed the bacmid clone that had not been recombined.

4. Packaging of recombinant baculovirus

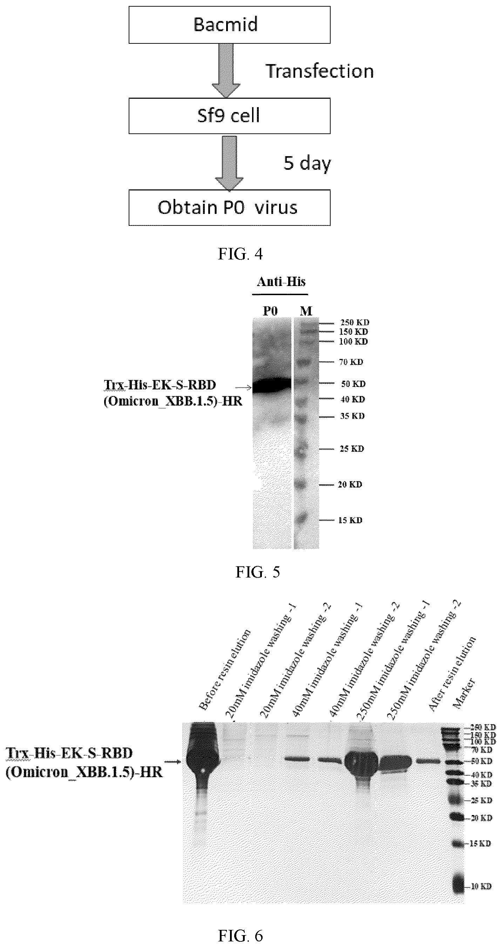

The recombinant bacmid was transfected into sf9 insect cells, and P0-passage recombinant baculovirus was harvested after 5 days. The packaging and amplification flowchart of the recombinant baculovirus were as shown in FIG. 4.

5. Expression and verification of target protein

The expression of the target protein was simultaneously taken place during the amplification of the above-mentioned baculovirus. While before removing the tag of the target protein, His tag was included, and therefore we used the WB experiment of Anti-His to verify the expression of the recombinant protein. The verification result showed that an obvious stripe was observed between 40 KD-55 KD Marker stripes, and had a size matching with that of the Trx-His-EK-S-RBD(Omicron_XBB.1.5)-HR protein, indicating the successful amplification of baculovirus and successful expression of the target protein. The testing result was as shown in FIG. 5.

6. Purification and identification of target protein

The P0-passage recombinant baculovirus was infected with sf9 cells, a cell culture fluid was harvested after 3 days, and the verification for the protein purification was carried out through Ni-affinity chromatography packing. The result was as shown in FIG. 6. The target protein was mainly eluted under 40 mM and 250 mM imidazoles, and the target protein with a high purity might be obtained after elution.

7. Verification for removing tag of EK restriction enzyme cutting

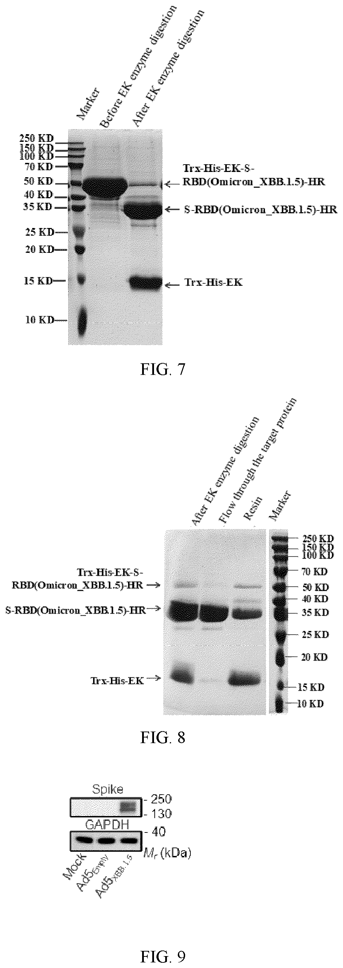

The target protein eluate is concentrated and adjusted to a concentration of 1 mg/ml, EK enzyme was added, and after enzyme digestion at 18 °C for 14 h, it was identified by SDS-PAGE gel electrophoresis. The result was as shown in FIG. 7. The result showed that the EK enzyme could cut the Trx-His-EK (the amino acid sequence at the EK restriction enzyme cutting site) tag from the target protein.

8. Removal verification for the removed tag

The sample after EK restriction enzyme cutting was reversely hung for 10 min by using the Ni-affinity chromatography packing, and the sample was eluted by using 250 mM imidazole after flowing through. The Trx-His-EK-S-RBD(Omicron_XBB.1.5)-HR protein (uncut and complete protein) and S-RBD(Omicron_XBB.1.5)-HR protein were separated from the cut Trx-His-EK tag. The result was as shown in FIG. 8. The results had shown that after the Trx-His-EK tag was removed, the removal could be performed by flow-through mode and subsequent purification by Ni-affinity chromatography filler, thereby obtaining S-RBD (Omicron_XBB.1.5)-HR protein without any tag. The amino acid sequence of the protein was shown in SEQ ID NO. 5.

Through the aforementioned recombinant protein construction method, based on the S protein RBD of SARS-CoV-2 Omicron_BA.5, XBB.1.5 or the S protein RBD-HR amino acid sequences of SARS-CoV-2 mutant strains, the applicants had designed protein constructs as specified in SEQ ID NO.8, SEQ ID NO.12, and SEQ ID NO.16, the ultimately expressed protein amino acid sequences were shown in SEQ ID NO.1, SEQ ID NO.3, and SEQ ID NO.5 respectively, the expressed recombinant proteins were formulated with adjuvants to prepare recombinant protein vaccines respectively: RBD$_{XBB.1.5}$-HR (SEQ ID NO.5), RBD$_{XBB.1.5}$ (SEQ ID NO.3), and RBD$_{BA.5}$-HR (SEQ ID NO.1), which were subsequently used for animal immunization studies and further research.

**Embodiment 2 Construction and Preparation of Recombinant Adenovirus Vaccine**

1. SARS-CoV-2 Omicron_BA. 5 and SARS-CoV-2 Omicron_BA.5, Omicron_XBB.1.5 S Protein Gene Sequences Optimization and Synthesis

Based on the mutation sites of the new coronavirus Omicron_BA.5 and Omicron_XBB.1.5 provided on the website https://covariants.org/, the nucleotide sequences of the S proteins of each SARS-CoV-2 variant were obtained, and at the same time, the signal peptide of each S protein mutant was retained and the codons are optimized. Subsequently, the S protein genes of each variant were synthesized according to the mutation and optimized sequences.
The synthesized SARS-CoV-2 Omicron_BA.5 and Omicron_XBB.1.5 S protein genes were shown in SEQ ID NO.23 and SEQ ID NO.21.

2. Packaging of Recombinant Adenovirus Novel Coronavirus Vaccine

During the gene synthesis process, after the synthesized product was cloned into the pDC316 vector using a recombinant cloning strategy, a shuttle plasmid (pDC316-S) could be obtained. The pDC316-S containing the S genes of the SARS-CoV-2 Omicron_BA.5 and Omicron_XBB.1.5 variants constructed above were co-transfected with the backbone plasmid pBHGlox_E1,3Cre of the AdMax adenovirus system into HEK293 cells for packaging of the recombinant adenovirus. The process was as follows:

1) Inoculated $8 \times 10^5$ HEK293A cells per well into a six-well plate with high-glucose DMEM + 10% FBS medium, cultured them overnight in a 37°C cell culture incubator containing 5% $CO_2$.
2) The next day, replaced the medium with high-glucose DMEM supplemented with 2% FBS, followed by co-transfection of HEK293A cells with the backbone plasmid (pBHGlox_E1,3Cre) and shuttle plasmid using Lipofectamine 3000. The specific steps were as follows: took $4\mu g$ of backbone plasmid and $2\mu g$ of shuttle plasmid from each transfection well, diluted with $125\mu L$ Opti-MEM medium, and then added $12\mu L$ P3000 reagent; took another 1.5ml EP tube, diluted $7.5\mu L$ lipofectamine3000 with $125\mu L$ Opti-MEM medium; mixed the diluted plasmid and diluted lipofectamine3000 in a 1:1 ratio, incubated at room temperature for 10-15 minutes, and then added to the cells, continued to culture the cells, and subcultured them in 25cm$^2$ cell culture flasks after the cells grew full, observed the signs of viral cytopathic effect (CPE) every day, when the cells grew full of the bottom of the flask, transfered them to 75cm$^2$ cell culture flasks until the cells showed obvious plaques, and harvested the virus when most of the cells were diseased and fell off from the bottom.
3) Collected the virus-containing cell culture, centrifuged at 1200rpm for 3 minutes, aspirated the virus-containing supernatant, resuspended the cell pellet with 1/10 culture volume of virus-containing supernatant, alternately placed in -80°C refrigerator and 37°C water bath, froze and thawed three times repeatedly. Centrifuged at 3000rpm for 20 minutes, collected the virus-containing supernatant, and combined it with the above-mentioned virus-containing supernatant, which was the virus seed of adenovirus vaccine.
4) Took $50\mu L$ of the vaccine candidate strain virus liquid, added $2\mu L$ of proteinase K, digested at 50°C for 30min to release the viral genome, used this as a template to PCR amplify the S gene sequence, and sequenced and identified the PCR product after electrophoresis gel recovery.

Denaturation: 95°C, 10min; denaturation: 95°C, 10s; annealing: 64°C, 30s; extension: 72°C, 2min; extension: 72°C, 5min; cycle number, 40 times; PCR amplification primers were as follows:

pDC516-F1: ACACGTCAATGGGAAGTGAAA(SEQ ID NO.24)
pDC516-R1: GCTAGACGATCCAGACATGAT(SEQ ID NO.25)

3. Amplification of Recombinant Adenovirus Novel Coronavirus Vaccine

The identified correct recombinant adenovirus vaccine strains were amplified step by step in 293 cells. The specific process was as follows: added 293 cells that were 80%-90% confluent at MOI=10, collected the virus culture after most of cells became round, and prepared the master virus seed bank and working virus seed bank according to the above-mentioned repeated freezing and thawing method. Used cell factories or bioreactors to scale up the recombinant adenovirus vaccine, and collected the virus culture after most of the cells were diseased. The process of amplifying cells and viruses in the bioreactor was as follows: First, added 3-5 g/L Cytodex1 microcarriers to the bioreactor and sterilized, then, added cell culture medium to the bioreactor, once the operating conditions were stabilized at 37°C, pH 7.0, DO 50%, and 50rpm, digested and harvested HEK293 cells amplified from the cell factory, and inoculated them into the bioreactor at a seeding density of $1.0\text{-}5.0\times10^5$ cells/ml, supplemented the cell culture medium to a total volume of 5L, maintained the bioreactor cell culture conditions at 37°C, 30-50rpm, pH7.15-7.25, and DO 30-50%, took daily samples to monitor glucose concentration, cell density and cell morphology on the microcarriers; inoculated the bioreactor with the recombinant adenovirus vaccine seed stock at an MOI of 5-30 when the cell density in the bioreactor reached $1.0\text{-}5.0\times10^6$, took daily samples to monitor glucose concentration after inoculation, measured the viral titer in the culture supernatant and cell pellet, and observed the morphology of cells on the microcarriers; stopped the culture when most cells had detached from the microcarriers, added virus lysis buffer to the bioreactor at a final concentration of 0.05%-1% (v/v) Tween 20, performed lysis at 37°C for 2-4 hours, then collected the viral solution.

4. Purification of Recombinant Adenovirus Vaccine

The collected virus was purified by cesium chloride ultracentrifugation or ion exchange chromatography, and the specific process was as follows:

(1) Purification of Adenovirus Vaccine by Cesium Chloride Ultracentrifugation

Centrifuged the collected viral culture at 1200g for 10 minutes and aspirated the virus-containing supernatant, resuspended the cell pellet in virus-containing supernatant at 1/10 of the original culture volume, performed three freeze-thaw cycles using a - 80°C freezer and 37°C water bath, centrifuged at 3000rpm for 10-20 minutes and collected the supernatant. Concentrated the virus-containing supernatant 10-fold using a 100K-300K ultrafiltration membrane; prepared a 1.4g/ml cesium chloride solution (by mixing 53g of cesium chloride with 87ml 10mM Tris-HCl, PH 7.9), prepared the 1.2g/ml cesium chloride solution (by mixing 26.8g of cesium chloride with 92 ml of 10 mM Tris-HCl (pH 7.9); slowly added 8ml of 1.4g/ml cesium chloride solution into the ultracentrifuge tube, followed by gently adding 6ml of 1.2g/ml cesium chloride solution, finally, added 20ml of virus-containing supernatant on top of the discontinuous gradient, balanced the tube, then centrifuged at $100000\times g$ and 4°C for 90 minutes; after centrifugation, used a syringe to extract the blue virus band, dialyzed to remove cesium chloride, and then stored the purified virus solution at -80°C.

(2) Purification of Adenovirus by Ion Exchange Chromatography

Collected the viral culture and lysed it using 0.05%-1% Tween 20 at 37°C for 2-4 hours, clarified the lysed culture by filtering through 1.2$\mu$m and 0.45$\mu$m capsule filters, concentrated the sample 10-fold using a tangential flow filtration membrane with a molecular weight cutoff of 100-300 kD, washed the concentrate with 10 volumes of washing buffer (50 mM Tris-HCl, 2 mM MgCl2, 0-500 mM NaCl, pH 8.0), and collected the washed sample; added nuclease to the washed sample at a final concentration of 10-50U/ml, digested at 37°C for 1-3 hours, then, performed anion exchange chromatography using resins such as Q Sepharose XL, Source 30Q, or Source 15Q, and the detailed procedure was as follows: Balanced the column with equilibration buffer at a flow rate of 20ml/min for 5 column volumes, after equilibration, loaded the sample at a flow rate of 10ml/min, and once loading was complete, continued equilibrating with equilibration buffer until the conductivity stabilized; eluted the sample using a linear gradient from 100% low-salt buffer to 100% high-salt buffer over 10 column volumes at a flow rate of 10 ml/min, and collected the fractions corresponding to each elution peak; after elution, regenerated the column with 2M NaCl buffer for 5-10 column volumes at a flow rate of 20ml/min. Collected the virus peak, then performed buffer exchange on the eluted virus sample by dialysis or tangential flow filtration (using the buffer composed of 10mM Tris, 10mM Na-PO$_4$, 150mM NaCl, 2mM MgCl$_2$, 2% sucrose, 0.15% glycerol, and 0.02% Tween 80, pH7.6).

Filled the purified adenovirus directly into containers, and stored at -20°C protected from light.

5. Identification of Recombinant Adenovirus Vaccines

We had firstly expressed the full-length spike glycoproteins of SARS-CoV-2 mutation variant Omicron BA.5 and Omicron XBB.1.5 using human replication-deficient Ad5 adenovirus vectors and detected the expression level of the spike protein in 293T cells 48 hours after infection with Ad5$_{XBB.1.5}$ and Ad5$_{BA.5}$ recombinant adenoviruses by Western blot. As shown in FIG. 9, the adenovirus Ad5$_{XBB.1.5}$ could induce high levels of Spike protein expression after infecting 293T cells, while empty adenovirus control Ad5 Empty could not, proving that our recombinant adenovirus vaccine had been successfully prepared.

Among them, the adenovirus vaccines prepared by Embodiment 1 were defined as Ad5$_{XBB.1.5}$ (amino acid sequence SEQ ID NO.20, nucleotide sequence SEQ ID NO.21) and Ad5 $_{BA.5}$ (amino acid sequence SEQ ID NO.22, nucleotide sequence SEQ ID NO.23) recombinant adenoviruses.

The following experiments proved the effects of the recombinant protein vaccine and adenovirus vaccine prepared by the present invention.

**Embodiment 3: Animal preparation, mice immunization and sample collection**

1. Female NIH mice aged 6-8 weeks were purchased from Charles River Laboratories, and the mice were raised in an environment without special pathogens of national key laboratory for biotherapy in Sichuan University. After the recombinant protein stock solution was diluted with PBS buffer and mixed with an equal volume ratio of adjuvant aluminum or MF59 in a total volume of 50 $\mu$l, the mice were immunized three times on days 0, 14, and 28, with 10 $\mu$g protein/mouse, and the blood was collected 7 days after the three immunizations to detect neutralizing antibodies and evaluate the immune effect of the vaccine.

2. Female BALB/c mice aged 6-8 weeks were purchased from Charles River Laboratories, and the mice were raised in an environment without special pathogens of national key laboratory for biotherapy in Sichuan University. To prepare the two-component nasal spray vaccine, $2.5\times10^9$ VP (low dose) or $5\times10^9$ VP (high dose) of Ad5$_{XBB.1.5}$ was mixed with 10 $\mu$g RBD $_{XBB.1.5}$-HR in a total volume of 50 $\mu$l to prepare low-dose or high-dose two-component vaccines respectively. BALB/c mice were immunized three times with (1) low and (2) high doses of Ad5$_{XBB.1.5}$ alone, (3) low and (4) high doses of the two-component (Ad5$_{XBB.1.5}$+RBD $_{XBB.1.5}$-HR) vaccine, (5) 10 $\mu$g of RBD $_{XBB.1.5}$-HR protein alone or (6) $5\times10^9$ VP of Ad5Empty mixed with RBD $_{XBB.1.5}$- HR protein and delivered intranasally, with an interval of 28 days. To avoid excessive fluid inflow into the lung tissue, the mice received two intranasal immunizations in one day, 25 $\mu$l each time, with an interval of more than 3 hours.

The preparation and immunization scheme of other two-component vaccines Ad5$_{BA.5}$+RBD $_{BA.5}$-HR, Ad5$_{BA.5}$+RBD $_{XBB.1.5}$-HR, and Ad5$_{XBB.1.5}$+RBD $_{BA.5}$-HR were similar to those described above, and the recombinant proteins in the single-component, two-component or three-component vaccines used for immunization contained an adjuvant MF59 of equal volume to the recombinant proteins.

The three-component vaccine consisting of $5\times10^9$ VP of Ad5$_{XBB.1.5}$, 3.3$\mu$g of RBD $_{BA.5}$-HR and 6.6$\mu$g of RBD $_{XBB.1.5}$-HR was administered intranasally with the same immunization schedule. The serum samples were collected at weeks 3, 7 and 11 of the vaccinated animals to determine binding and neutralizing antibody responses. To further evaluate the mucosal immunity induced by the vaccine, the mice were euthanized on the 21st day after the third booster vaccination to collect BALF and lung tissues.

**Embodiment 4:** Detection of antibodies by an enzyme-linked immunosorbent assay (ELISA)

To detect RBD specificities IgG and IgA of novel coronavirus, a 96-well plate (NUNC-MaxiSorp, Thermo Fisher Scientific) was coated with 1 $\mu$g/mL of recombinant RBD protein in a carbonate-bicarbonate buffer solution, and overnight at 4°C. In the next day, the plate was washed for three times with 1$\times$PBS containing 0.1% Tween-20 (PBST), and then sealed for one hour with PBST containing 1%BSA. The continuously diluted serum, broncho-pulmonary lavage or nose swab in the dilution buffer solution were added to the well (100 $\mu$l/well). After the incubation was performed for one hour at 37°C, the plate was washed for three times, then diluted horse radish peroxidase (HRP) conjugated goat anti-mouse IgG (1:10000, southern biotech, Cat : 0107-05), HRP-conjugated goat anti-mouse IgG (1:5000), HRP-conjugated goat anti-rabbit IgG, HRP-conjugated goat anti-rabbit IgG, HRP-conjugated goat anti-human IgG (southern biotech, Cat: 62-8420) or HRP-conjugated goat anti-human IgA (southern biotech, Cat: 2050-05). Then, after the incubation was performed for one hour at 37°C, the plate was further washed for three times, and developed for 10 min with 3, 3', 5 ,5'-tetramethylbenzidine (TMB) at room temperature. The reaction was stopped with 1M $H_2SO_4$. Finally, the absorbance at 450 nm was measured on a microwell plate reader (Spectramax ABS, Molecular Devices). A terminal titer was defined as the highest inverse dilution of serum with the absorbance greater than or equal to 2.1 times of negative control serum value.

The results were shown in FIGs.. 10-14, after the immunization, the preparation of recombinant adenovirus Ad5 combined with recombinant RBD induced stronger serum and alveolar lavage fluid anti-RBD-specific binding antibodies than Ad5 adenovirus alone, indicating that the adenovirus combined with recombinant subunit preparations could produce stronger humoral immune protection. In addition, the simple RBD nasal immunization could not induce the strong blood-binding antibodies, but after combining with empty Ad5$_{Empty}$ or recombinant adenovirus Ad5, its immunogenicity could be significantly improved, which proved that the adenovirus could be used as an adjuvant of recombinant subunits to enhance the immunogenicity of protein antigens.

**Embodiment 5 Novel Coronavirus Pseudovirus Neutralization Test**

In order to detect the titer of neutralizing antibodies in serum and BALF samples, the pseudovirus neutralization test had been performed above. The pseudovirus of luciferase was expressed, including prototype, $\delta$ and Omicron subline (BA.2.75, BA.5, BF.7, BQ.1, BQ.1.1, XBB, XBB.1.5, XBB.1.16 and the like), purchased from Genomeditech Company.

Briefly, the inactivated serum and BALF samples (56°C for 30min) were diluted threefold, ranging from 30 to 65610, and then incubated with an equal volume of pseudovirus at different dilutions at 37°C for 1 h. Then, $1.2\times10^4$ HEK-293T cells expressing human ACE2 receptor (293T/ACE2) were added to each well and incubated at 37°C for 48 h to express luciferase. Finally, the supernate was removed, then a lysis reagent (Beyotime, RG005) with a luciferase substrate was added, and a multi-mode a microwell plate reader (PerkinElmer, USA) was used to measure the luminous quantity in the 293T/ACE2 cell. 50% neutralizing rate of the pseudovirus was measured and calculated with GraphPad Prism 8.0.2. A positive control group only included cells and viruses, a negative control group only included cells, and a sample group included cells, samples and viruses. A neutralizing percentage was calculated by the following formulas:

Neutralization (%) = (positive sample-sample to be measured/positive sample-negative sample) $\times$ 100%

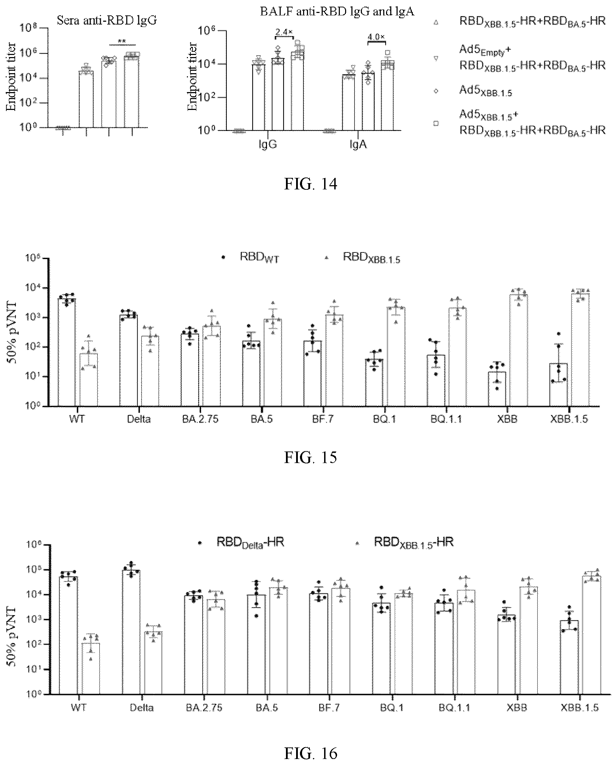

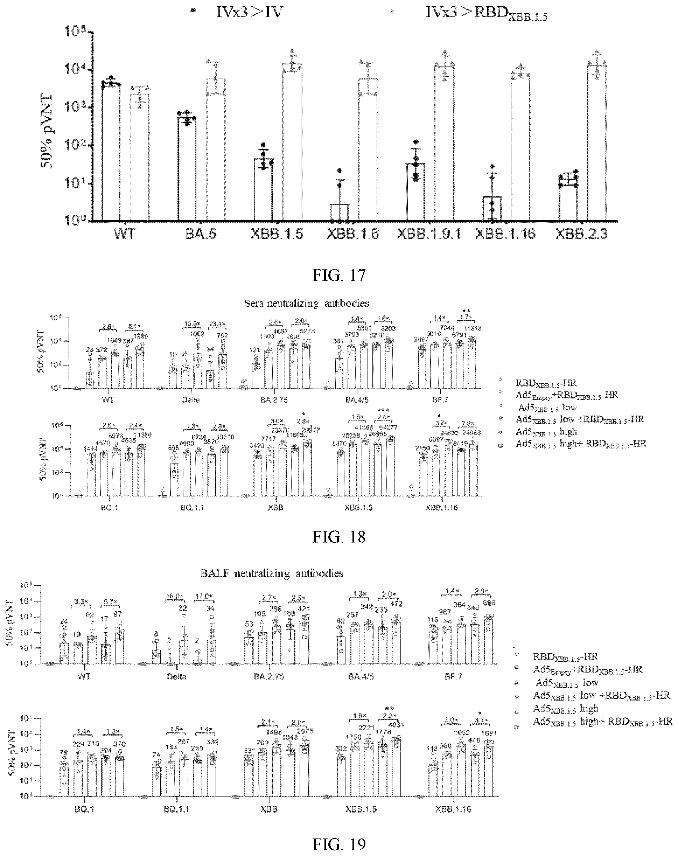

As shown in FIGs. 15-16, after three injections of pure protein vaccines (recombinant $RBD_{XBB.1.5}$-HR and recombinant $RBD_{XBB.1.5}$), the strong serum neutralizing antibodies against WT, Delta, BA.2.75, BF.7, BA.5, BQ.1, BQ.1.1, XBB and XBB.1.5 viruses were produced for the mice, and the neutralizing antibodies induced by MF59 adjuvant were significantly better than those induced by aluminum adjuvant ($RB_{DWT}$ and $RBD_{Delta}$-HR in FIG. 15 are recombinant proteins prepared using the protein construction method described in Embodiment 1, based on the publicly available RBD sequence of the wild-type SARS-CoV-2 S protein and the RBD-HR sequence of the Delta variant respectively); FIG. 17 showed that after three intramuscular immunizations with 25U per dose of inactivated vaccine on days 0, 14, and 42, sequential booster vaccinations with either inactivated vaccine (produced by Sinopharm) or $RBD_{XBB.1.5}$ were administered via intramuscular injection at 84 days after the last immunization, with an immunization dose of $10\mu g$ per dose, serum was collected 14 days post-booster to measure pseudovirus-neutralizing antibodies, mice vaccinated with the $RBD_{XBB.1.5}$ vaccine generated robust serum neutralizing antibodies against WT, BA.5, XBB.1.5, XBB.1.6, XBB.1.16, XBB.1.9.1, and XBB.2.3 viruses, with particularly strong efficacy against the recently dominant XBB subvariants and other circulating strains.

The bivalent vaccine formulation combining adenovirus and recombinant RBD protein induced stronger neutralizing antibodies in both serum and bronchoalveolar lavage fluid compared to adenovirus alone, demonstrating that the combination of adenovirus and recombinant subunit could provide stronger systemic and mucosal neutralizing protection, effectively preventing viral infection; as shown in FIGs. 18-20, $Ad5_{XBB.1.5}$ + $RBD_{XBB.1.5}$-HR had elicited high levels of serum neutralizing antibodies against WT, Delta, BA.2.75, BF.7, BA.5, BQ.1, BQ.1.1, XBB, XBB.1.5, and XBB.1.16, suggesting that the combination of an Omicron S-protein adenoviral vaccine and S-RBD protein could generate stronger immune protection against Omicron variants.

**Embodiment 6 Novel Coronavirus Live Virus Neutralization Test**

The neutralization antibodies in serum samples from mice vaccinated with the $Ad5_{XBB.1.5}$ + $RBD_{XBB.1.5}$-HR bivalent vaccine against live ancestral and mutated novel coronaviruses were measured through authentic virus neutralization assays. Each group of diluted serum was mixed with the live novel coronavirus at 50% tissue culture infection dose (TCID50). After incubation at 37°C for 1 hour, the mixture was added to a 96-well microplate pre-seeded with Vero E6 cells ($5\times10^4$/well) and incubated for 72 hours. Cell pathogenic effect (CPF) was measured with a microscope, and the titer of the neutralizing antibodies causing the EC50 (50% neutralizing) to be inhibited in the immune serum was calculated.

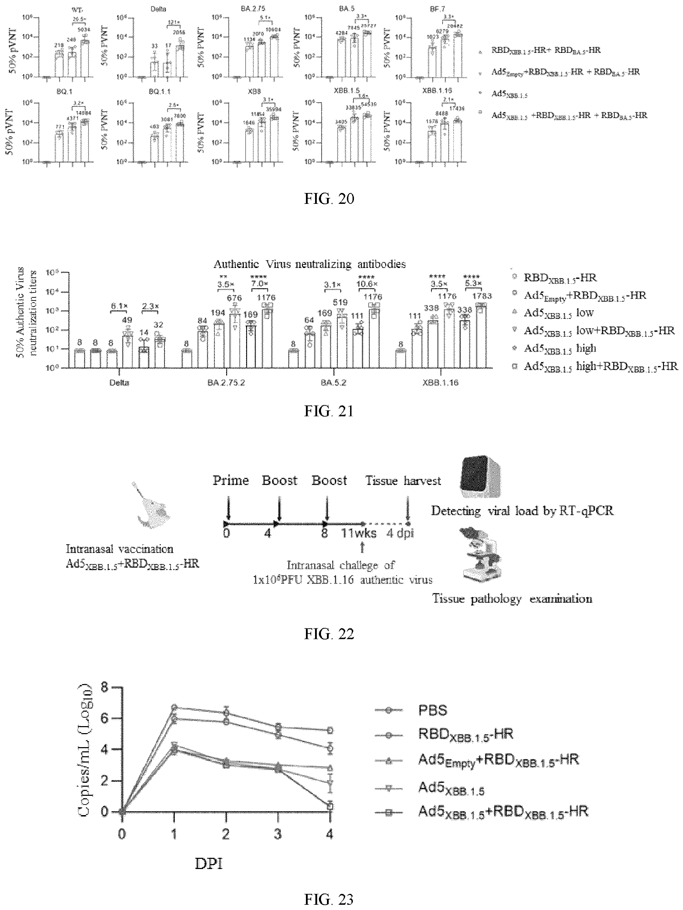

The results, as shown in FIG. 21, indicated that the serum from mice intranasally administered with the bivalent $Ad5_{XBB.1.5}$ + $RBD_{XBB.1.5}$-HR vaccine exhibited neutralizing antibody titers exceeding 1000 against live Delta and Omicron variants, particularly the recently prevalent XBB.1.16 strain, and were significantly higher than those observed with intranasal administration of adenovirus or protein alone.

**Embodiment 7 Attack of the Novel Coronavirus XBB.1.16 Variant**

On days 0, 28, and 56, the BALB/c mice were intranasally immunized with three times of the $Ad5_{XBB.1.5}$ vaccine, alone or in combination with two or three components. The mice immunized with PBS or $Ad5_{Empty}$ served as controls, with n=6 mice per group. On the 21st day after the final vaccination, all mice were intranasally challenged with live SARS-CoV-2 XBB.1.16 Omicron variant ($1\times10^6$ PFU). The mouse were subjected to euthanasia and their tissues were collected on day 4 after infection. Pathological changes of lung tissues were observed through hematoxylin eosin stain. The viral load in nasal turbinate, trachea, and lung tissue samples was assessed by reverse transcription quantitative polymerase chain reaction (RT-qPCR) targeting viral genomic RNA (gRNA), and the primer sequences were 5'-GACCCCAAAATCAGC

GAAAT-3' (forward) (SEQ ID NO.26) and 5'-TCTGGTTACTGCCCAGTTGAATCTG-3' (reverse) (SEQ ID NO.27), with the probe sequence 5'-FAM-ACGCCGCATTACGTTTGGTGGGACC-BHQ1-3' (SEQ ID NO.28). All procedures involving mouse challenge with the novel coronavirus Omicron variant were reviewed and approved by Institutional Animal Care and Use Committee of Institute of Medical Biology, Chinese Academy of Medical Sciences, and were carried out in ABSL-4 facilities of Kunming National High-level Biosafety Primate Research Center.

BALB/c mice were intranasally immunized three times with $Ad5_{XBB.1.5}$ and the bivalent vaccine ($Ad5_{XBB.1.5}$+RBD $_{XBB.1.5}$-HR). The mice treated with PBS, naked RBD $_{XBB.1.5}$-HR , Ad5Empty + RBD $_{XBB.1.5}$-HR served as controls. The BALB/c mice immunized and then challenged with $1\times10^6$PFU of live SARS-CoV-2 XBB.1.16 Omicron variant were used to evaluate changes in viral load in throat swabs after SARS-CoV-2 infection; on day 4 post-infection, the nasal turbinate, trachea and lung tissues were collected to measure levels of gRNA and sgRNA using RT-qPCR; the histopathological changes in lung tissues following Omicron challenge were also observed.

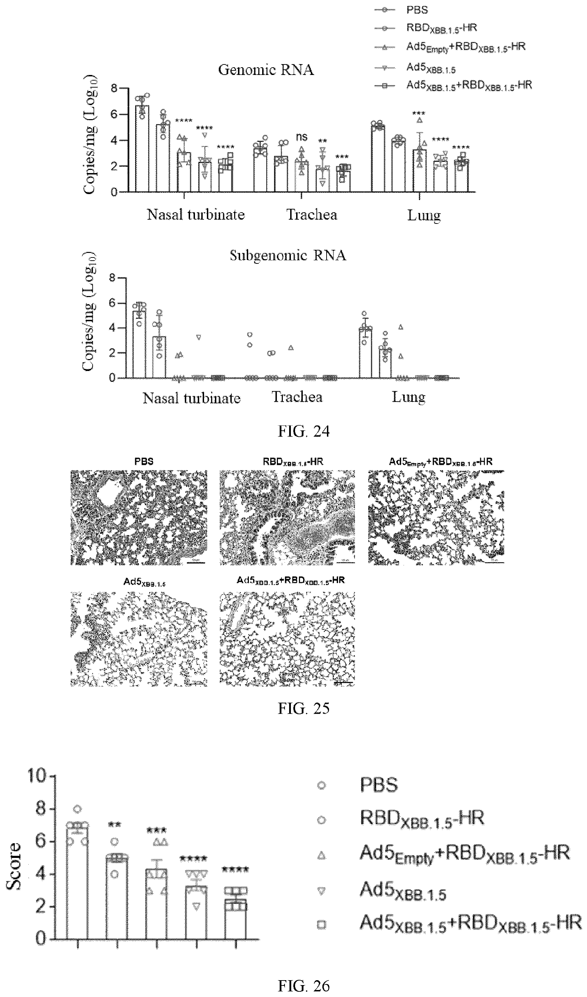

As shown in FIGs. 22-26, the mice treated with immune serum induced by the $Ad5_{XBB.1.5}$ ($5\times10^9$ VP/mouse) + RBD $_{XBB.1.5}$-HR (10 μg) combination vaccine exhibited nearly complete clearance of viral load in throat swabs, no viral RNA was detected in the nasal turbinate, trachea, or lung tissues, the lung histology appeared normal, the alveolar structures were intact and there was no significant inflammation; in contrast, the PBS and other control groups showed significant viral loads in throat swab samples and elevated levels of viral RNA in the nasal turbinate, trachea, and lung tissues, and the histopathological examination revealed mild pathological changes, including multifocal consolidation areas, slight thickening of alveolar septa, and alveolar congestion; additionally, the small foci of inflammation composed of macrophages, neutrophils and lymphocytes were occasionally observed near small blood vessels. The results demonstrated that the $Ad5_{XBB.1.5}$+RBD $_{XBB.1.5}$-HR bivalent vaccine provides complete protection against infection by the XBB.1.16 Omicron variant.

## Claims

1. A protein against infections by SARS-CoV-2 Omicron variant XBB and subvariants thereof, wherein: it contains an amino acid sequence selected from any one of SEQ ID NO. 1 to SEQ ID NO. 7.

2. A precursor of the protein according to claim 1, wherein: a signal peptide and/or a protein tag is connected to the protein as described.

3. The protein precursor according to claim 2, wherein: the protein tag is selected from at least one of the following: a histidine tag, a thioredoxin tag, a glutathione-S-transferase tag, a ubiquitin-like modifier tag, a maltose-binding protein tag, a c-Myc protein tag, an Avi protein tag, or a nitrogen assimilation substance A protein tag.

4. The protein precursor according to claim 3, wherein a protease recognition region for excising the protein tag is also linked to the protein against the infection by the SARS-CoV-2 Omicron variant XBB and subvariants thereof; and preferably, the protease is selected from at least one of an enterokinase, a TEV protease, a thrombin, a blood coagulation factor Xa, a carboxypeptidase A, and a rhinovirus 3c protease.

5. The protein precursor according to any one of claims 2-4, wherein: the amino acid sequence of the precursor is selected from at least one of SEQ ID NO.8, SEQ ID NO.10, SEQ ID NO.12, SEQ ID NO.14, SEQ ID NO.16, SEQ ID NO.18, and SEQ ID NO.20.

6. A polynucleotide, wherein: it encodes the protein according to claim 1 or the precursor according to any one of claims 2-5.

7. The polynucleotide according to claim 6, wherein: the nucleotide sequence is selected from at least one of SEQ ID NO.9, SEQ ID NO.11, SEQ ID NO.13, SEQ ID NO.15, SEQ ID NO.17, SEQ ID NO.19 and SEQ ID NO.21.

8. A recombinant vector, wherein: comprising the polynucleotide according to claim 6 or 7.

9. The recombinant vector according to claim 8, wherein the recombinant vector is at least one of an insect baculovirus expression vector, a mammalian cell expression vector, an escherichia coli expression vector and a yeast expression vector; preferably, the insect baculovirus expression vector is pFastBac1; the escherichia coli expression vector is pET32a; the yeast expression vector is pPICZaA; the mammalian cell expression vector is a CHO cell expression vector; and further preferably, the CHO cell expression vector is pTT5 or FTP-002.

10. A host cell, wherein: comprising the recombinant vector according to claim 8 or 9.

11. The host cell according to claim 10, wherein: the host cell is at least one of an insect cells, a mammalian cells, an Escherichia coli, and a yeast; preferably, the insect cell is selected from at least one of a sf9 cells, a sf21 cells, and a Hi5 cells; the mammalian cell is a CHO cell.

12. A preparation method for the protein precursor according to any one of claims 2 to 5 or the protein according to claims 1, wherein: comprising the following steps: culturing the host cell according to claim 10 or 11, such that the host cell expresses the protein or the precursor, then recovering the protein, to obtain the protein and the precursor,.

13. A protein composition for resisting infections caused by SARS-CoV-2 Omicron variants and subvariants, wherein: it includes any two or more of BA.5 sequence-1, BA.5 sequence-2, XBB.1.5 sequence-1, XBB.1.5 sequence-2, XBB.1.5 sequence-3, XBB.1.5 sequence-4, and S-XBB.1.5 proteins.

14. The protein composition according to claim 13, wherein: the protein composition comprises one of BA.5 sequence-1 and BA.5 sequence-2, any one of XBB.1.5 sequence-1, XBB.1.5 sequence-2, XBB.1.5 sequence-3 and XBB.1.5 sequence-4 and at least two combinations of S-XBB.1.5 protein; preferably, the protein composition comprises a combination of one of BA.5 sequence-1 and BA.5 sequence-2, and any one of XBB.1.5 sequence-3 and XBB.1.5 sequence-4 with S-XBB.1.5 protein.

15. A recombinant protein vaccine for preventing and/or treating infections by SARS-CoV-2 Omicron variants and subvariants thereof, wherein: it contains the protein according to claim 1, the precursor according to any one of claims 2-5 and/or the protein composition according to any one of claims 13-14, and the pharmaceutically acceptable excipients or auxiliary ingredients.

16. The recombinant protein vaccine according to claim 15, wherein the adjuvant component is an immunologic adjuvant; and preferably, the immunologic adjuvant is selected from at least one of the following: a squalene-based oil-in-water emulsion, an aluminum salt, a calcium salt, a phytosaponin, a phytopolysaccharide, a monophosphate lipid A, a muramyl dipeptide, a muramyl tripeptide, a bacterial toxin, a GM-CSF cytokine, a lipid and cationic liposomal material.

17. The recombinant protein vaccine according to claim 15 or 16, wherein at least one of the following is satisfied: the squalene-based oil-in-water emulsion being MF59; the aluminum salt being selected from at least one of aluminum hydroxide and alum; the calcium salt being tricalcium phosphate; the phytosaponin being either QS-21 or ISCOM; the phytopolysaccharide being astragalus polysaccharide; the bacterial toxin being selected from at least one of recombinant cholera toxin and diphtheria toxin; the lipid being selected from at least one of the following: phosphatidylethanolamine, phosphatidylcholine, cholesterol, dioleoylphosphatidylethanolamine; the cationic liposome material being selected from at least one of the following: (2,3-dioleoyloxypropyl) trimethyl ammonium chloride, N-[1-(2,3-dioleoyl chloride) propyl]-N,N,N-trimethylamine chloride, cationic cholesterol, dimethyl-2,3-dioleyloxypropyl-2-(2-spermidinecarboxamido)ethyl ammonium trifluoroacetate, trimethyldodecylammonium bromide, trimethyltetradecylammonium bromide, trimethylhexadecylammonium bromide, dimethyldioctadecylammonium bromide, and CpG ODN.

18. An adenovirus vector vaccine for preventing and/or treating infections by SARS-CoV-2 Omicron variants and subvariants thereof, wherein: it constructs a recombinant vector containing a polynucleotide encoding the amino acid sequence of the protein according to claim 1, the precursor according to any one of claims 2-5 or the protein composition according to any one of claims 13-14.

19. The adenovirus vector vaccine according to claim 18, **characterized in that** the nucleotide sequence of the polynucleotide is selected from at least one of SEQ ID NO.9, SEQ ID NO.11, SEQ ID NO.13, SEQ ID NO.15, SEQ ID NO.17, SEQ ID NO.19, and SEQ ID NO.21; more preferably, the polynucleotide sequence contained in the adenovirus vector is selected from the polynucleotide for constructing S-XBB.1.5, as shown in SEQ ID NO.21.

20. The adenovirus vector vaccine according to claim 18, wherein: the adenovirus vector is selected from at least one of the following: an adenovirus, an Ankara vaccinia virus, and an adeno-associated virus; preferably, it is selected from a human type 5, type 35 or type 26 or/and chimpanzee AdC68 or AdC7 replication-deficient adenovirus; more preferably, it is selected from a human type 5 replication-deficient adenovirus with combined deletion of E1 and E3.

21. The adenovirus vector vaccine according to any one of claims 18-19, wherein: it also includes a pharmaceutically acceptable adjuvant, vector, diluent or excipient.

22. A preparation method for the adenovirus in the adenovirus vector vaccine according to any one of claims 18-21, comprising the following steps: constructing a shuttle plasmid vector of the polynucleotide; then transfecting the constructed shuttle plasmid vector into host cells together with the backbone plasmid, and culturing the host cells; obtaining the replication-deficient recombinant adenovirus, and then expanding the culture and purifying.

23. The recombinant protein vaccine according to any one of claims 15 to 17 and the adenovirus vector vaccine according to any one of claims 18 to 21 are intradermal or subcutaneous injection preparations, intramuscular injection preparations, intravenous injection preparations, oral or nasal spray preparations; preferably, the vaccine is an intramuscular injection preparation and a nasal spray preparation.

24. A vaccine composition for treating and/or preventing infections by SARS-CoV-2 Omicron variants and subvariants thereof, wherein: it is a compound preparation containing the recombinant protein vaccine in any one of claims 15-17 and the adenovirus vector vaccine in any one of claims 18-21 as active ingredients.

25. A combined drug for treating and/or preventing infections by SARS-CoV-2 Omicron variants and subvariants thereof, wherein: comprising the recombinant protein vaccine in any one of claims 15-17 and the adenovirus vector vaccine in any one of claims 18-21, which are administered separately or simultaneously.

26. The composition according to claim 24 or the combined drug according to claim 25, wherein: comprising a combination or combined drug of recombinant protein vaccine 1, and/or recombinant protein vaccine 2 and adenovirus vector vaccine; preferably, the recombinant protein vaccine 1 contains at least one amino acid sequence of BA.5 sequence-1, BA.5 sequence-2 protein or precursor; the recombinant protein vaccine 2 contains at least one amino acid sequence of XBB.1.5 sequence-1, XBB.1.5 sequence-2, XBB.1.5 sequence-3, XBB.1.5 sequence-4 protein or precursor; the adenovirus vector vaccine contains the polynucleotide sequence of S-XBB.1.5.

27. The composition or the combined drug according to claim 26, wherein: the amino acid sequence contained in the recombinant protein vaccine 1 is selected from at least one of SEQ ID NO.1, SEQ ID NO.2, SEQ ID NO.8 and SEQ ID NO.10; the amino acid sequence contained in the recombinant protein vaccine 2 is selected from at least one of SEQ ID NO.3 to SEQ ID NO.6, SEQ ID NO.12, SEQ ID NO.14, SEQ ID NO.16 and SEQ ID NO.18; preferably, it is selected from at least one of SEQ ID NO.5, SEQ ID NO.6, SEQ ID NO.16 and SEQ ID NO.18; the adenovirus vector vaccine contains the polynucleotide sequence as shown in SEQ ID NO.21.

28. The composition or the combined drug according to claim 27, wherein: the composition or the combined drug is an intradermal or subcutaneous injection preparation, an intramuscular injection preparation, an intravenous injection preparation, an oral or nasal spray preparation; preferably, the vaccine is an intramuscular injection preparation and a nasal spray preparation.

29. The protein according to claim 1, the precursor in any of claims 2-5, the protein composition in any of claims 13-14, the recombinant protein vaccine in any of claims 15-17, the adenoviral vector vaccine in any of claims 18-21, and the vaccine composition or the combined drug in any of claims 24-28 is applicable in the preparation of pharmaceutical agents for treating and/or preventing infections or pathogenesis caused by SARS-CoV-2 Omicron variants and subvariants thereof.

30. A vaccine composition for treating and/or preventing infections caused by SARS-CoV-2 Omicron variants and subvariants thereof, wherein: it contains the recombinant protein vaccine and the adenoviral vector vaccine in any of claims 15-17; the amino acid sequence of the recombinant protein vaccine is selected from at least one of SEQ ID NO.1 to SEQ ID NO.7 and SEQ ID NO.8 to SEQ ID NO.20; the nucleotide sequence for the antigen of the adenoviral vector vaccine is selected from SEQ ID NO.23.

31. A combined drug for treating and/or preventing infections by SARS-CoV-2 Omicron variants and subvariants thereof, wherein: the recombinant protein vaccine and adenovirus vector vaccine in any one of claims 15-17 is administered separately or simultaneously; the amino acid sequence of the recombinant protein vaccine is selected from at least one of SEQ ID NO.1 to SEQ ID NO.7 and SEQ ID NO.8 to SEQ ID NO.20; the nucleotide sequence for the antigen of the adenoviral vector vaccine is selected from SEQ ID NO.23.

FIG. 1

FIG. 2

FIG. 3

FIG. 4

FIG. 5

FIG. 6

FIG. 7

FIG. 8

FIG. 9

FIG. 10

FIG. 11

FIG. 12

FIG. 13

FIG. 14

FIG. 15

FIG. 16

FIG. 17

FIG. 18

FIG. 19

FIG. 20

FIG. 21

FIG. 22

FIG. 23

FIG. 24

FIG. 25

FIG. 26

## INTERNATIONAL SEARCH REPORT

| International application No. |
| --- |
| **PCT/CN2023/113170** |

| A. | CLASSIFICATION OF SUBJECT MATTER |
| --- | --- |
| | C07K14/165(2006.01)i; C07K19/00(2006.01)i; C12N15/50(2006.01)i; A61K39/215(2006.01)i; A61P31/14(2006.01)i |

According to International Patent Classification (IPC) or to both national classification and IPC

| B. | FIELDS SEARCHED |
| --- | --- |

Minimum documentation searched (classification system followed by classification symbols)

   IPC:C07K,C12N,A61P

Documentation searched other than minimum documentation to the extent that such documents are included in the fields searched

Electronic data base consulted during the international search (name of data base and, where practicable, search terms used)

   DWPI, VEN, CNABS, CNTXT, CNKI, PubMed, GenBank, EBI, STN, WPABSC, WPABS, ENTXT, ENTXTC, Wanfang Database, ISI web of Knowledge: 新冠, 新型冠状, COVID-19, SARS-Cov-2, 奥秘克戎, Omicron, XBB.1.5, BA.5, 疫苗, 腺病毒, 信号肽, RBD, 申请人, 发明人, SEQ ID NO: 1-23

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | JOST, M. et al. "Surface Glycoprotein, Partial[Severe Acute Respiratory Syndrome Coronavirus 2]" *NCBI GenBank WDE90833.1*, 17 February 2023 (2023-02-17), origin | 1-31 |
| X | HOWARD, D. et al. "Surface Glycoprotein[Severe Acute Respiratory Syndrome Coronavirus2]" *NCBI GenBank: WCC38909.1*, 26 January 2023 (2023-01-26), origin | 1-31 |
| X | GELLES, R. L. et al. "Early Estimates of Bivalent mRNA Booster Dose Vaccine Effectiveness in Preventing Symptomatic SARS-CoV-2 Infection Attributable to Omicron BA.5- and XBB/XBB.1.5-Related Sublineages Among Immunocompetent Adults-Increasing Community Access to Testing Program, United States, December 2022-January 2023" *Morbidity and Mortality Weekly Report*, Vol. 72, No. 5, 03 February 2023 (2023-02-03), pages 119-124 | 13-31 |

| ✓ Further documents are listed in the continuation of Box C. | ✓ See patent family annex. |
| --- | --- |

| | * | Special categories of cited documents: | "T" | later document published after the international filing date or priority date and not in conflict with the application but cited to understand the principle or theory underlying the invention |
| --- | --- | --- | --- | --- |
| | "A" | document defining the general state of the art which is not considered to be of particular relevance | | |
| | "D" | document cited by the applicant in the international application | "X" | document of particular relevance; the claimed invention cannot be considered novel or cannot be considered to involve an inventive step when the document is taken alone |
| | "E" | earlier application or patent but published on or after the international filing date | | |
| | "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| | "O" | document referring to an oral disclosure, use, exhibition or other means | "&" | document member of the same patent family |
| | "P" | document published prior to the international filing date but later than the priority date claimed | | |

| Date of the actual completion of the international search | Date of mailing of the international search report |
| --- | --- |
| **09 October 2023** | **20 October 2023** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
|---|
| **PCT/CN2023/113170** |

**C. DOCUMENTS CONSIDERED TO BE RELEVANT**

| Category* | Citation of document, with indication, where appropriate, of the relevant passages | Relevant to claim No. |
|---|---|---|
| Y | KURHADE, C. et al. "Low Neutralization of SARS-CoV-2 Omicron BA.2.75.2, BQ.1.1 and XBB.1 by Parental mRNA Vaccine or a BA.5 Bivalent Booster."<br>*Nature Medicine*, Vol. 29, 06 December 2022 (2022-12-06),<br>pages 344-347 | 13-31 |
| Y | DHAMA, K. et al. "SARS-CoV-2 Emerging Omicron Subvariants with a Special Focus on BF.7 and XBB.1.5 Recently Posing Fears of Rising Cases Amid Ongoing COVID-19 Pandemic."<br>*Journal of Experimental Biology and Agricultural Sciences.*,<br>Vol. 10, No. 6, 31 December 2022 (2022-12-31),<br>pages 1215-1221 | 13-31 |
| Y | WO 2023026207 A1 (TRANSLATIONAL HEALTH SCIENCE AND TECHNOLOGY INSTITUTE et al.) 02 March 2023 (2023-03-02)<br>claims 1-11 | 13-31 |
| A | CN 113480619 A (SHENZHEN YHLO BIOTECH CO., LTD.) 08 October 2021 (2021-10-08)<br>entire document | 1-31 |
| A | US 2021393769 A1 (GILL DAVINDER) 23 December 2021 (2021-12-23)<br>entire document | 1-31 |
| A | WO 2021159040 A2 (MODERNATX, INC.) 12 August 2021 (2021-08-12)<br>entire document | 1-31 |
| A | WO 2022266511 A1 (ELIXIRGEN THERAPEUTICS, INC.) 22 December 2022 (2022-12-22)<br>entire document | 1-31 |
| A | WO 2023019309 A1 (MONASH UNIVERSITY) 23 February 2023 (2023-02-23)<br>entire document | 1-31 |
| A | CN 112300251 A (SICHUAN UNIVERSITY) 02 February 2021 (2021-02-02)<br>entire document | 1-31 |
| A | CN 113173977 A (JIANGSU KUNLI BIO-PHARMACY CO., LTD.) 27 July 2021 (2021-07-27)<br>entire document | 1-31 |

Form PCT/ISA/210 (second sheet) (July 2022)

41

## INTERNATIONAL SEARCH REPORT

| International application No. |
|---|
| **PCT/CN2023/113170** |

**Box No. I     Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑ forming part of the international application as filed.

    b.  ☐ furnished subsequent to the international filing date for the purposes of international search (Rule 13*ter*.1(a)),

        ☐ accompanied by a statement to the effect that the sequence listing does not go beyond the disclosure in the international application as filed.

2.  ☐ With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

**INTERNATIONAL SEARCH REPORT**
Information on patent family members

International application No.

**PCT/CN2023/113170**

| Patent document cited in search report | | | Publication date (day/month/year) | Patent family member(s) | | | Publication date (day/month/year) |
|---|---|---|---|---|---|---|---|
| WO | 2023026207 | A1 | 02 March 2023 | None | | | |
| CN | 113480619 | A | 08 October 2021 | None | | | |
| US | 2021393769 | A1 | 23 December 2021 | None | | | |
| WO | 2021159040 | A2 | 12 August 2021 | AU | 2021215938 | A1 | 01 September 2022 |
| | | | | KR | 20220140528 | A | 18 October 2022 |
| | | | | JP | 2023513544 | A | 31 March 2023 |
| | | | | IL | 295377 | A | 01 October 2022 |
| | | | | CA | 3170150 | A1 | 12 August 2021 |
| | | | | BR | 112022015565 | A2 | 27 September 2022 |
| | | | | EP | 4100052 | A2 | 14 December 2022 |
| | | | | WO | 2021159040 | A3 | 04 November 2021 |
| | | | | WO | 2021159040 | A9 | 25 November 2021 |
| WO | 2022266511 | A1 | 22 December 2022 | WO | 2022266511 | A9 | 13 July 2023 |
| WO | 2023019309 | A1 | 23 February 2023 | None | | | |
| CN | 112300251 | A | 02 February 2021 | US | 2023233665 | A1 | 27 July 2023 |
| | | | | JP | 2023522795 | A | 31 May 2023 |
| | | | | EP | 4112648 | A1 | 04 January 2023 |
| | | | | WO | 2021169255 | A1 | 02 September 2021 |
| | | | | CA | 3186989 | A1 | 02 September 2021 |
| | | | | BR | 112022021758 | A2 | 17 January 2023 |
| CN | 113173977 | A | 27 July 2021 | None | | | |

Form PCT/ISA/210 (patent family annex) (July 2022)